(19) Europäisches Patentamt
European Patent Office
Office européen des brevets

(11) EP 4 101 830 A1

(12) EUROPEAN PATENT APPLICATION

(43) Date of publication:
**14.12.2022 Bulletin 2022/50**

(21) Application number: **21178716.3**

(22) Date of filing: **10.06.2021**

(51) International Patent Classification (IPC):
***C07C 25/18*** *(2006.01)* ***C07C 43/225*** *(2006.01)*
***C08F 2/48*** *(2006.01)* ***C09D 163/00*** *(2006.01)*
*G03F 7/004 (2006.01)* *G03F 7/029 (2006.01)*
*B41C 1/10 (2006.01)*

(52) Cooperative Patent Classification (CPC):
**C07C 25/18; C07C 43/225; C08F 2/50;**
**C09D 163/00; G03F 7/0045; G03F 7/029;**
B41C 1/1008

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **FEW Chemicals GmbH**
**06766 Bitterfeld-Wolfen (DE)**

(72) Inventors:
- **Keil, Dietmar**
  **06766 Bitterfeld-Wolfen (DE)**
- **Gehrmann, Philipp**
  **04317 Leipzig (DE)**
- **Licha, Kai**
  **14612 Falkensee (DE)**
- **Lerch, Frank**
  **83229 Aschau im Chiemgau (DE)**
- **Werner, Saija**
  **37197 Hattorf am Harz (DE)**
- **Simpson, Christopher**
  **37520 Osterode am Harz (DE)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

# (54) NOVEL DIARYLIODONIUM SALT MIXTURES AS LOW MOLECULAR WEIGHT PHOTOINITIATORS WITH MINIMIZED CRYSTALLIZATION BEHAVIOR AND ELEVATED SOLUBILITY

(57) The invention relates to novel diaryliodonium salt mixtures as low molecular weight photoinitiators with minimized crystallization behavior and elevated solubility, advantageous methods for their synthesis and manufacturing, as well as their application in compositions for coatings, varnishes, layers, and printing plates.

EP 4 101 830 A1

## Description

### Summary of the invention

The invention relates to novel diaryliodonium salt mixtures as low molecular weight photoinitiators with minimized crystallization behavior and elevated solubility, advantageous methods for their synthesis and manufacturing, as well as their application in compositions for coatings, varnishes, layers, and printing plates.

Diaryliodonium salts were reported in 1864 for the first time (Berichte der Deutschen Chemischen Gesellschaft; Hartmann, Meyer, 1894, 27, 426). Ever since, this structural class has found entry into numerous technical applications, mostly as initiator in photochemical processes. Diaryliodonium salts according to general formula I with $R^1 = R^2$ and $R^1 \neq R^2$ are employed as photoradical generators (PRG) as well as photoacid generators (PAG) for mechanisms of photocuring in applications of coatings, varnishes, and materials which are cured by polymerization and/or crosslinking.

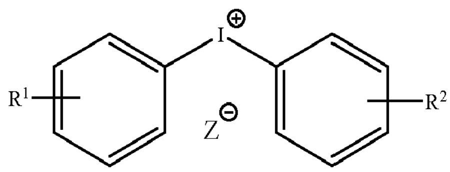

**Formula (A)**

Especially in mechanisms of curing by light, thereby inducing photopolymerization, it is mandatory to employ as little as possible of such compounds in a polymerizable mixture, to avoid crystallization and other negative effects to the quality of the desired products. This is particularly important in the area of CtP (Computer-to-plate offset printing), where printing plates need to have highest homogeneity of their surfaces. Thus, it is important to enable highest possible efficacy of photoinitiators, which is achievable by generating as many as possible initiating molecules (e.g. radicals in radical photopolymerization) per mass unit (such as grams of applied substance). Thus, this invention employs alkyliodonium salts of low molecular weight, corresponding to short chain alkyl substituents (1 to 7 carbon atoms, branched or unbranched) for $R_1$ and $R_2$ in the general formula I.

It is well known that with decreasing molecular weight and corresponding increased impact of the cationic charge to the chemical structure of diaryliodonium salts, the solubility in nonpolar solvents will become lower. In turn, melting points and ability to crystallize will be increased, which may cause unwanted effects of crystallization in polymeric materials, coatings, layers, and surfaces.

It is well known to the person skilled in the art that the variation of alkyl substituents has impact on crystallization and solubility. The state of the art teaches that longer chain alkyl substituents reduce ability to crystallize and enhance solubility. Higher molecular weight, however, is disadvantageous related to the fact, that smaller numbers of effective radicals are formed per mass unit of applied iodonium salt in a photopolymerizable composition.

As known for the skilled artisan, melting points and crystallization ability of pure chemical entities can be lowered by mixing such pure compounds (principle of eutectic systems; see Smith, William F.; Hashemi, Javad (2006), Foundations of Materials Science and Engineering (4th ed.), McGraw-Hill, ISBN 978-0-07-295358-9). A mixture, composed by mechanic mixing two single compounds, will usually exhibit a lower melting point than each of the single compounds. For the application of diaryliodonium salts as photoinitiators, this has been applied for the manufacturing of printing plates (WO2017131959).

According to this invention, the surprisingly found solution to this limitation is based on the utilization of impurities being part of the diaryliodonium salts. This is achieved by iodonium salt mixtures, as further detailed below, yielding to improved physicochemical properties while maintaining low molecular weight. This is achieved by performing a synthesis of diaryliodoniums salts with more than one purified starting material of alkyl substituted aryls, yielding a synthesis mixture composed of a plurality of both asymmetric and symmetric iodonium salt structures. The relative constituents are formed based on principles of the synthesis reaction mechanism (see detailed description).

For example, the use of three differently substituted arylalkyl (R-Ar) aromatic compounds serve as starting materials to enable an electrophilic substitution at the Ar moiety, leading to a mixture of diaryliodonium salts, as outlined below in detail.

### Technological field of the invention

The invention concerns novel diaryliodonium salt mixtures useful as photoinitiators with improved properties, advantageous methods for their preparation, as well as their application especially for the manufacturing of coatings, varnishes, layers, and printing plates.

**State of the art**

Diaryliodonium structures are obtained by two or three synthetic steps, as known to a person skilled in the art. In a first step in the reaction mechanism, an iodoaryl moiety is oxidized to an iodine (III)-complex. Secondly, a ligand exchange with aryl or aryl-metal-organyl yields a diaryliodonium cation (with counter anion depending on the reagents used). The counter anion can finally be exchanged by a desired other anion.

Furthermore, symmetric diaryliodonium salts are readily obtained by the use of preformed hypervalent iodine(III) reagents (see Figure 2), such as condensing iodosylarenes (ArI=O) or iodoxyarenes ($ArIO_2$) to a diaryliodonium structure (H. J. Lucas, Org. Synth. 1942, 22, 52). Such mechanisms are ineffective, and less economic due to low yields, and thus remained meaningless for several decades (R. B. Sandin, Chem. Rev. 1943, 32, 249). Optimized pathways were developed by BERINGER *et al* by utilizing hypervalent iodo moieties such as iodosylaryls, (diacetoxyiodo)aryls and iodoxyaryls in the presence of strong acids (F. M. Beringer et al. , J. Am. Chem. Soc. 1959, 81, 342 & 1953, 75, 2705; J. V. Crivello, J. H. W. Lam, J. Org. Chem. 1978, 43, 3055). This enabled increased yields by supporting the expected mechanism of aromatic electrophilic substitution ($S_E$). In particular, acetic anhydride / trifluoroacetic acid supported the reaction with electron-rich aryls, whereas sulfuric acid worked best for electron-deficient aryls (F. M. Beringer et al., J. Am. Chem. Soc. 1958, 80, 4279; Y. Yamada, M. Okawara, Bull. Chem. Soc. Jpn. 1972, 45, 2515). The aromatic electrophilic substitution of an aryl (Ar-H) to an iodo(III)-entity is selective and follows the known mechanistic rules. Activated aryls (+M and/or +I-effect of a residue R at the aryl Ar-R) direct into their 2- and 4-position (ortho/para position), with preference for the 4-position due to steric hindrance in the 2-position, usually increasing with sterically more demanding substituents at the aryl (Ar-R).

KOSER *et al.* developed a method enabling selective access to defined non-symmetric iodonium salts by applying hydroxy(tosyloxy)iodobenzene (Koser's reagent) with arylsilanes and then also arylstannates (G. F. Koser et al., J. Org. Chem. 1980, 45, 1543; C. S. Carman et al., J. Org. Chem. 1983, 48, 2534; A. J. Margida et al., J. Org. Chem. 1984, 49, 3643). A further improvement has been to date, that oxidation and ligand exchange can be done in one process step, i.e. they are directly received from aryls and iodoaryls or even molecular iodine $I_2$ (H. Tohma et al., Angew. Chem. 2004, 116, 3679; Angew. Chem. Int. Ed. 2004, 43, 3595; T. Dohi et al.; Angew. Chem. 2005, 117, 6349; Angew. Chem. Int. Ed. 2005, 44, 6193; M. Ochiai et al., J. Am. Chem. Soc. 2005, 127, 12244). OLOFSSON *et al.* published an efficient one-pot-method starting from diaryliodoniumtriflates obtained from aryliodides and aryl moieties, with *meta*-chloroperbenzoic acid (mCPBA), which generates in situ (diacyloxy)iodoarenes (M. Bielawski et al., Chem. Commun. 2007, 2521; Adv. Synth. Catal. 2007, 349, 2610). Due to good solubilities of the oxidizing and acidic reagents, high yields with low excess of reagents were reported.

Basis of the present invention is the method of BERINGER *et al.* (F. M. Beringer et al. , J. Am. Chem. Soc. 1959, 81, 342 & 1953, 75, 2705) towards symmetric diaryliodonium salt bromides, obtained by reaction of monofunctional arenes (Ar-R) in the presence of potassium iodate, acetic acid and acetic anhydride and sulfuric acid. Here, reactive iodosylsulfate is formed *in-situ,* followed by treatment with aqueous KBr solution and recrystallization from methanol. The avoidance of pre-synthesized, expensive aryliodides makes this method economic, and further allows to identify inventive iodonium salt mixtures with surprising behavior, when applying different, multiple aryl compounds in the reaction.

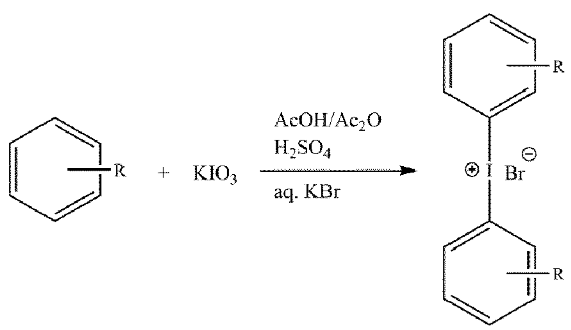


Diaryliodonium structures have been applied already back in the early 1980s as radical initiators in the area of polymerization under UV and thermal conditions, such as for the manufacturing of printing plates and coatings. This went along with substituting more expensive and toxic Pd-, Hg- und Pb-based organyls as catalysts. In the area of printing technologies, diaryliodonium salts have emerged as crucial components in the chemical composition of printing plates for Computer-to-plate offset methods (CtP; Brömme et al. Chem. Eng. Technol. 2016, 39, No. 1, 13-25).

To decrease the tendency of forming crystals in layers, and for the improvement of solubility in recipes for coatings, diaryliodonium salts are often applied as mixtures generated by previously synthesized, pure single compounds (WO 2017/11959 A1). This type of physically generated mixtures leads to decreased ability to crystallize and better

solubility (based on known principles of eutectic mixing), but has often shown to be not sufficient for long time storage of coatings and manufacturing of printing plates.

The synthesis of diaryliodonium salt mixtures, according to the method in Fig. 2. (Behringer et al.), by using different arylalkanes in the starting reaction solution (R-Ar) is described in WO2010128649A1, (or EP2428501 and JP5485583B2 within this patent family). According to all embodiments of the disclosure, aryl chains of 8 to 20 carbon atoms are essential to suppress the tendency to from crystals, and in particular, solubility of the mixture is improved by increasing the length of the alkyl chain substituents in iodonium salts. The inventors of the present application demonstrate that an increased solubility of the respective iodonium mixtures impedes their isolation unfavorably, with difficulties to obtain solid materials, often yielding oils. This is a drawback regarding economic manufacture and purification. Furthermore, the described iodonium salts mixtures were isolated with bromides and iodide as counter anion, which makes them less effective as photoinitiators in photopolymerization.

The current drawbacks and deficiencies of the relevant prior art lead to the invention of novel iodonium salt mixtures with improved properties for a broad technological field, and to methods of an efficient synthesis and manufacture.

**Detailed description of the invention**

The invention relates to novel diaryliodonium salt mixtures according to **Formula (A)** as low molecular weight photoinitiators with minimized crystallization behavior and elevated solubility, advantageous methods for their synthesis and manufacturing, as well as their application in compositions for coatings, varnishes, layers, and printing plates.

In the context of the invention, diaryliodonium salt mixtures shall be understood as a material composed of a plurality of different chemical diaryliodonium structures, which are formed together in one synthetic procedure, thereby yielding a synthetic mixture. As opposed to this, technically mixed diaryliodonium salts are defined derived from a number of single and pure compounds, each previously and independently synthesized and purified, followed by their weighting and mixing together, thereby generating a technical mixture.

The synthetic mixtures according to the invention comprise three or more different diaryliodonium cations each neutralized with an anion (thereby forming an iodonium salt), wherein the aryl moieties are substituted with alkyl or alkoxy substituents, giving a diaryliodonium salt mixture constituted of at least $x = \frac{n(n+1)}{2}$ (n = number of different structures of substituted aryls R-Ar) different compounds when only assuming the reaction mechanism of 4-substitution (para-substitution).

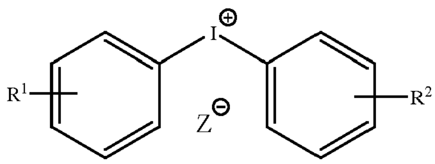

**General formula (A)**

Two or more different Aryls (R-Ar) are used for the synthesis, yielding a diaryliodonium salt mixture of **general formula (A),** constituted of at least three defined single structures, wherein at least one of these single structures comprises $R^1$ and $R^2$ being different substituents.

Considering rules of electrophilic aromatic substitution, known to the skilled artisan, the number of components in the mixture can be enhanced considering 2- and 4-positions of the carbon-iodine bond formation relative to the alkyl position in R-Ar (ortho and para-position), which is mechanistically favored with +M and/or +I-activating substituents. The 2-position (ortho-position) is disfavored, due to steric hindrance. Thus, a mixture will contain para/para-structures as major components, followed by ortho/para-structures and ortho/ortho-structures in different ratios depending on the steric demands of the R substituents.

For example, the use of three differently substituted arylalkyl (R-Ar) aromatic compounds as suitable starting material for conducting an electrophilic substitution at the Ar moiety, leads to a mixture of diaryliodonium salts. This mixture is constituted by six different single structures, under the assumption that each aromatic moiety performs one isomeric type of carbon-iodine bond formation (usually in the preferred *para-* or 4-position relative to the alkyl position). Accordingly, the number x of different structural entities of diaryliodonium cations in the mixture is $x = \frac{n(n+1)}{2} = 6$ (n = number of different structures of substituted aryls R-Ar = 3). With each additional event of an *ortho-* or 2-substitution, the number of possible entities is increased further, thereby enhancing the complexity of the mixture and improving

desired properties according to the invention. For three differently substituted arylalkyl (R-Ar), all possible structures formed are illustrated in **Figure 1.** Generally, the number of possible all structures y including o/o, o/p and p/p-isomers, can be calculated by the equation $y = 2x + n^2$.

Accordingly, the composition of a diaryliodonium salt mixture can be described by the following chemical formulae (I) to (X), which can be formed in the synthesis as components of the mixture to a different relative extent.

| | R1 + R2 | R1 + R1 | R2 + R2 |
|---|---|---|---|
| p/p | 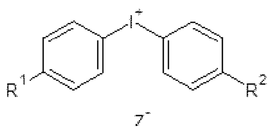 | 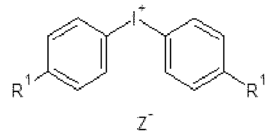 | 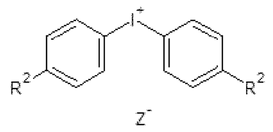 |
| | (I) | (V) | (VIII) |
| o/p | 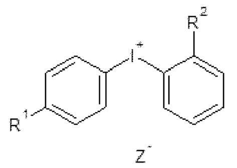 | 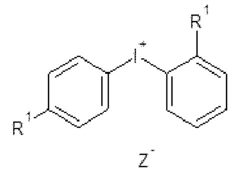 | 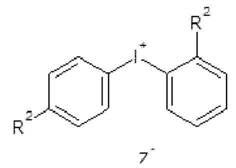 |
| | (II) | (VI) | (IX) |
| p/o | 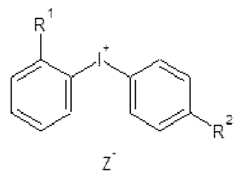 | = o/p | = o/p |
| | (III) | | |
| o/o | 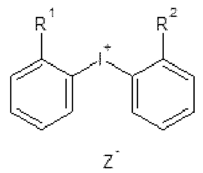 | 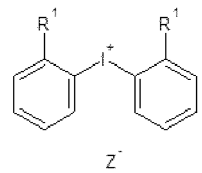 | 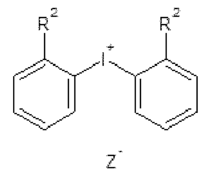 |
| | (IV) | (VII) | (X) |

The analysis of the composition obtained by the reaction procedures can be conducted by usual analytical methods (HPLC, mass spectroscopy, NMR). The relative composition can be quantified by HPLC, as known to the skilled person, e.g. by integrating all detected peaks. Mass spectrometry can qualitatively show existence of all possible molecular weight generated, independent of the o/p-substitution pattern.

Mixtures contain at least $x = \frac{n(n+1)}{2}$ different structures (with n = number of different structures of substituted aryls R-Ar in the starting mixture of the synthesis; $R_1 \neq R_2$) of formula (I) - (X). At least one of the iodonium compounds of the mixture is selected from unsymmetrical compounds (I) to (IV). Within the embodiment, diaryliodonium salt mixtures contain the structures (I), (V) and (VIII). That means, components include all possible symmetric and non-symmetric structures for each pair of $R^1$ and $R^2$, formed with the predominant para-substitution, with usually non-symmetric components in higher relative amount compared to the respective symmetric compounds with equal $R_1$ and $R_2$. Structures of type (I), (V) and (VIII) are based on para-substitution, as outlined above, being the predominant components in the mixture. However, the existence of ortho/para-based components in lower, and ortho/ortho-based components in even lower relative amount would enhance the complexity of the mixture, thereby further decreasing crystallinity and increasing solubility. We have demonstrated the existence of ortho/para-structures as part of the synthetic mixtures by qualitative H-NMR (See Figure 6 and 7). Therefore, preferred are mixtures containing at least one structure selected from an ortho/para-pattern of formula (II), (III), (VI) or (IX), or an ortho/ortho pattern of formula (IV), (VII) or (X), in addition to the predominant components of type (I), (V) and (VIII). Even more preferred are such mixtures, containing at least two

identified structures selected from an ortho/para-pattern of formula (II), (III), (VI) or (IX), or an ortho/ortho pattern of formula (IV), (VII) or (X).

The quantification of the relative amounts of components within the mixture can be conducted with techniques known to the skilled person, such as HPLC. We have shown by HPLC that three predominant components are detected, which are accompanied by smaller peaks of additional components. These exhibit the ortho/para-pattern of formula (II), (III), (VI) or (IX), or an ortho/ortho pattern of formula (IV), (VII) or (X), as illustrated in Figure 6 and 7. In combination with mass spectrometry (HPLC-MS) further characterization is possible. Thus, in another embodiment, a content of any detected ortho/para-pattern within formula (II), (III), (VI) or (IX) of above 0,1 % is preferred, more preferred above 1%, even more preferred above 2%.

Substituents $R^1$ and $R^2$ can be linear or branched $C_1$ to $C_7$-alkyl or alkoxy groups. Maximal efficacy by generated radicals per mass is achieved, when residues are as small as possible. According to the invention, diaryliodonium salt mixtures with linear or branched $C_1$ to $C_4$-alkyl or alkoxy groups lead to increased solubility and decreased melting points compared to pure compounds of similar molecular weight, and are thus preferred embodiment.

Accordingly, residues $R^1$ and $R^2$ in the diaryliodonium salt mixtures are selected from the group of t-butyl, s-butyl, n-butyl, i-propyl, n-propyl, ethyl, methyl, t-butyloxy, s-butyloxy, n-butyloxy, i-propyloxy, n-propyloxy, ethoxy and methoxy. Preferred are $R^1$ and $R^2$ in the diaryliodonium salt mixtures being selected from the group of t-butyl, s-butyl, n-butyl, i-propyl, n-propyl, ethyl, methyl, t-butyloxy, s-butyloxy, n-butyloxy, i-propyloxy, n-propyloxy, ethoxy and methoxy. More preferred are $R^1$ and $R^2$ being selected from the group methyl, ethyl, i-propyl, t-butyl, s-butyl, n-butyl and methoxy. Even more preferred are $R^1$ and $R^2$ being selected from the group methyl, ethyl, i-propyl, t-butyl, s-butyl, n-butyl.

It is well known to the skilled person, that R can be hydrogen (R = H) giving unsubstituted benzene as aryl. For reasons of safety and toxicology, this is not favored within the scope of the invention since products would generate free benzene in the application as photoinitiators.

Favorable properties comprise improved solubility in non-polar solvents or matrices and a decreased melting point (see data in examples). Decreasing melting points for diaryliodonium salts was reported to be possible only by long chain alkyl substituents (such as dodecyl), as taught by WO20100128649. This publication describes alkyl substituents of 8 to 20 carbon atoms. Confirmatory own work using > 8 carbon atoms showed substantial difficulties in the isolation of such diaryliodonium salt mixtures as solid materials, making purification disadvantageous. Thus, the technical feasibility for industrial manufacturing is limited.

Within the embodiment of the invention, it was found that all diaryliodonium salt mixtures with substituents R not exceeding 7 carbon atoms could be isolated as solid materials for different counteranions, while exhibiting decreased melting points and enhanced solubility in non-polar solvents at the same time (see Examples for methods and analytical data). The combination of lowest possible molecular weight of the diaryliodonium cations of the mixture with the ability to obtain the products as solid materials is a surprisingly found feature of the invention. Thus, preferred are diaryliodonium salt mixtures wherein no single diaryliodonium salt cation exceeds a molecular weight of 394 g/mol, more preferred 380 g/mol, even more preferred 352 g/mol.

A mixture based on $R^1/R^2$ being t-butyl/i-propyl exhibits a melting point of 135 - 140°C (compound **1**), compared to the pure, single component iodonium salt ($R^1=R^2$ = t-butyl) showing a melting point of 145 - 148°C (data for the respective tetraphenylborate salts). Prior art gives similarly decreased melting points for only long chains of R = $C_{10}$-$C_{13}$. Thus, efficacy of photoinitiation, when given by radicals per mass, is higher for low molecular weight mixtures of the embodiment. Furthermore, the solubility in nonpolar solvents (such as ketones, ethers, esters) is improved. For instance, a mixture based on $R^1/R^2$ being t-butyl/i-propyl (compound 1) exhibits a solubility of 187 g / L, compared to the pure iodonium salt ($R^1=R^2$ = t-butyl) showing a solubility of 149 g / L (measured in MEK/dowanol 1:1).

$Z^-$ according to general formula I and the formulas (I) - (X) are the same or different counteranions. Anions according to the invention can be organic, organometallic or inorganic anions. Preferred are anions selected from the group of chloride, bromide, iodide, perchlorate, tosylate, dodecylphenylsulfonate, hydrogensulfate, hexafluorophosphate, tris(pentafluoroethyl)-trifluorophosphate, hexafluoroantimonate, bis(trifluoromethanesulfonyl)imide ($NTf_2$; $(CF_3SO_2)_2N^-$), tris(trifluoromethanesulfonyl)methide (triflide; $CTf_3$; $(CF_3SO_2)_3C^-$), tetrafluoroborate, tetraphenylborate, triphenylmonoalkylborate, tetrakis(pentafluorophenyl)borate, tetrakis(di(trifluoromethyl)phenyl)borate, tetrakis(4-chlorophenyl)borate, tetrakis(perfluoro-tert-butoxy)aluminate $[((CF_3)_3C\text{-}O)_4Al^-)]$. More preferred are anions selected from the group of hexafluorophosphate, tetraphenylborate, tetrakis(perfluoro-tert-butoxy)aluminate $[((CF_3)_3C\text{-}O)_4Al^-)]$.

Preferred are diaryliodonium salt mixtures containing one single type of counteranion for all different diaryliodonium cations.

Preferred diaryliodonium salt mixtures within the embodiment of formulas (I) - (X) are summarized in the following table with entries 1 - 60, each entry comprising a synthetic mixture containing the components of formula (I), (V) and (VIII). More preferred are entries 1, 2, 7, 8, 13, 14, 19, 20, 25, 26, 31, 32, 37, 38, 43, 44, 49, 50, 55, 56 as photoradical generators. Entries 3, 4, 9, 10, 15, 16, 19, 21, 22, 27, 28, 33, 34, 39, 40, 45, 46, 51, 52, 57, 58 are more preferred as photoacid generators. Most preferred are these diaryliodonium salt mixtures, wherein the obtained composition contains more than one additional component, even more preferred more than two additional components, selected from the

formula (II), (III), (IV), (VI), (VII), (IX), (X), as outlined above.

| Nr. | n | $R_1$ / $R_2$ | Anion |
|---|---|---|---|
| **1** | 2 | t-butyl / i-propyl | tetraphenylborate |
| **2** | 2 | t-butyl / i-propyl | triphenyl-monobutylborate |
| **3** | 2 | t-butyl / i-propyl | hexafluorophosphate |
| **4** | 2 | t-butyl / i-propyl | tetrakis(perfluoro-*tert*-butoxy)aluminate |
| **5** | 2 | t-butyl / i-propyl | tris(pentafluoroethyl)trifluorophosphate |
| **6** | 2 | t-butyl / i-propyl | bis(trifluoromethanesulfonyl)imide |
| **7** | 2 | t-butyl / n-butyl | tetraphenylborate |
| **8** | 2 | t-butyl / n-butyl | triphenyl-monobutylborate |
| **9** | 2 | t-butyl / n-butyl | hexafluorophosphate |
| **10** | 2 | t-butyl / n-butyl | tetrakis(perfluoro-*tert*-butoxy)aluminate |
| **11** | 2 | t-butyl / n-butyl | tris(pentafluoroethyl)trifluorophosphate |
| **12** | 2 | t-butyl / n-butyl | bis(trifluoromethanesulfonyl)imide |
| **13** | 2 | t-butyl / methyl | tetraphenylborate |
| **14** | 2 | t-butyl / methyl | triphenyl-monobutylborate |
| **15** | 2 | t-butyl / methyl | hexafluorophosphate |
| **16** | 2 | t-butyl / methyl | tetrakis(perfluoro-*tert*-butoxy)aluminate |
| **17** | 2 | t-butyl / methyl | tris(pentafluoroethyl)trifluorophosphate |
| **18** | 2 | t-butyl / methyl | bis(trifluoromethanesulfonyl)imide |
| **19** | 2 | s-butyl / n-butyl | tetraphenylborate |
| **20** | 2 | s-butyl / n-butyl | triphenyl-monobutylborate |
| **21** | 2 | s-butyl / n-butyl | hexafluorophosphate |
| **22** | 2 | s-butyl / n-butyl | tetrakis(perfluoro-*tert*-butoxy)aluminate |
| **23** | 2 | s-butyl / n-butyl | tris(pentafluoroethyl)trifluorophosphate |
| **24** | 2 | s-butyl / n-butyl | bis(trifluoromethanesulfonyl)imide |
| **25** | 2 | i-propyl / methyl | tetraphenylborate |
| **26** | 2 | i-propyl / methyl | triphenyl-monobutylborate |
| **27** | 2 | i-propyl / methyl | hexafluorophosphate |
| **28** | 2 | i-propyl / methyl | tetrakis(perfluoro-*tert*-butoxy)aluminate |
| **29** | 2 | i-propyl / methyl | tris(pentafluoroethyl)trifluorophosphate |
| **30** | 2 | i-propyl / methyl | bis(trifluoromethanesulfonyl)imide |
| **31** | 3 | t-butyl / i-propyl / methyl | tetraphenylborate |
| **32** | 3 | t-butyl / i-propyl / methyl | triphenyl-monobutylborate |
| **33** | 3 | t-butyl / i-propyl / methyl | hexafluorophosphate |
| **34** | 3 | t-butyl / i-propyl / methyl | tetrakis(perfluoro-*tert*-butoxy)aluminate |
| **35** | 3 | t-butyl / i-propyl / methyl | tris(pentafluoroethyl)trifluorophosphate |
| **36** | 3 | t-butyl / i-propyl / methyl | bis(trifluoromethanesulfonyl)imide |
| **37** | 3 | t-butyl / n-butyl / methyl | tetraphenylborate |

(continued)

| Nr. | n | $R_1$ / $R_2$ | Anion |
|---|---|---|---|
| **38** | 3 | t-butyl / n-butyl / methyl | triphenyl-monobutylborate |
| **39** | 3 | t-butyl / n-butyl / methyl | hexafluorophosphate |
| **40** | 3 | t-butyl / n-butyl / methyl | tetrakis(perfluoro-*tert*-butoxy)aluminate |
| **41** | 3 | t-butyl / n-butyl / methyl | tris(pentafluoroethyl)trifluorophosphate |
| **42** | 3 | t-butyl / n-butyl / methyl | bis(trifluoromethanesulfonyl)imide |
| **43** | 3 | t-butyl / i-propyl / methyl | tetraphenylborate |
| **44** | 3 | t-butyl / i-propyl / methyl | triphenyl-monobutylborate |
| **45** | 3 | t-butyl / i-propyl / methyl | hexafluorophosphate |
| **46** | 3 | t-butyl / i-propyl / methyl | tetrakis(perfluoro-*tert*-butoxy)aluminate |
| **47** | 3 | t-butyl / i-propyl / methyl | tris(pentafluoroethyl)trifluorophosphate |
| **48** | 3 | t-butyl / i-propyl / methyl | bis(trifluoromethanesulfonyl)imide |
| **49** | 3 | t-butyl / i-propyl / methoxy | tetraphenylborate |
| **50** | 3 | t-butyl / i-propyl / methoxy | triphenyl-monobutylborate |
| **51** | 3 | t-butyl / i-propyl / methoxy | hexafluorophosphate |
| **52** | 3 | t-butyl / i-propyl / methoxy | tetrakis(perfluoro-*tert*-butoxy)aluminate |
| **53** | 3 | t-butyl / i-propyl / methoxy | tris(pentafluoroethyl)trifluorophosphate |
| **54** | 3 | t-butyl / i-propyl / methoxy | bis(trifluoromethanesulfonyl)imide |
| **55** | 4 | t-butyl / i-propyl / ethyl / methyl | tetraphenylborate |
| **56** | 4 | t-butyl / i-propyl / ethyl / methyl | triphenyl-monobutylborate |
| **57** | 4 | t-butyl / i-propyl / ethyl / methyl | hexafluorophosphate |
| **58** | 4 | t-butyl / i-propyl / ethyl / methyl | tetrakis(perfluoro-*tert*-butoxy)aluminate |
| **59** | 4 | t-butyl / i-propyl / ethyl / methyl | tris(pentafluoroethyl)trifluorophosphate |
| **60** | 4 | t-butyl / i-propyl / ethyl / methyl | bis(trifluoromethanesulfonyl)imide |

**Methods of synthesis**

The cationic component in the diaryliodonium salt mixtures can be synthesized as published (see section state-of-the-art). The technical mixing of previously synthesized, pure iodonium salts of single structure is not scope of the embodiment, as defined above.

Within the scope of the invention, a preferred method to synthesize diaryliodonium salt mixtures is the mixing of a number of **n** different alkylaryls or alkoxyaryls with linear or branched alkyl or alkoxy substituents of up to 7 carbon atoms, and performing a reaction in the presence of an iodate salt, or periodate, or m-chloroperbenzoic acid (see examples), forming a salt with hydrogensulfate as counteranion. In more detail, the synthesis is accomplished by mixing two or more alkylaryls and/or alkoxyaryls together with alkalimetal iodate or alkalimetal periodate salt, in acetic anhydride, acetic acid and sulfuric acid, and performing the reaction under temperature control. The reaction solution is quenched with ice/water and the resulting solution is extracted with solvents, such as dichloromethane or chloroform. The organic phase can be further washed and dried. Precipitation with a suitable solvent yields the desired diaryliodonium salt mixtures with hydrogensulfate as counteranion. The final product with a desired counterion is obtained by using aqueous solutions of respective salts, yielding the diaryliodonium salt mixtures according to the invention as solid materials (see picture below).

A preferred method for the synthesis comprises the pathway described above, with performing the isolation of the diaryliodonium salt mixture with hydrogen sulfate as solid precipitate, thereby allowing an intermediate step of purification.

Preferred is such a method using alkylaryls or alkoxyaryls with substituents selected from the group comprising

methyl, ethyl, *i*-propyl, t-butyl, s-butyl, n-butyl and methoxy. Preferred aqueous solutions to convert the hydrogen sulfate-based solid intermediates into the desired products with respective counteranions, are those with KI, KBr, NaBPh4, Li{Al[OC($CF_3$)$_3$]$_4$} and $KPF_6$, yielding diaryliodonium salt mixtures with iodide, bromide, tetraphenylborate, hexafluorophosphate or tetrakisperfluoro-*tert*-butoxyaluminate as counteranion.

1.

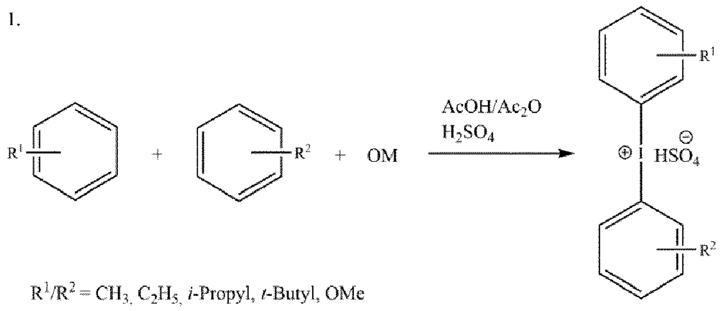


$R^1/R^2 = CH_3, C_2H_5,$ *i*-Propyl, *t*-Butyl, OMe

OM = $KIO_3$, $NaIO_3$, $NaIO_4$

2.

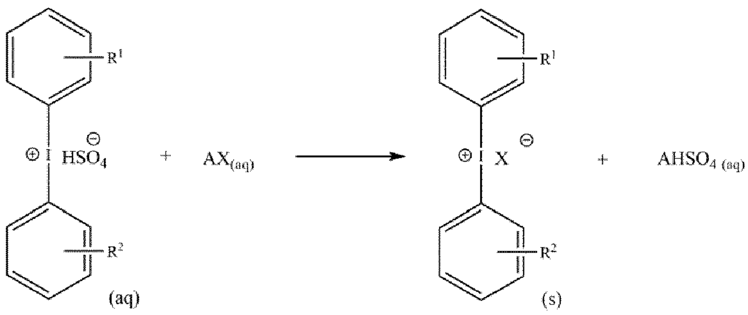

$A = Li^+, Na^+, K^+, NH_4^+$

$X = BPh_4^-, PF_6^-, OTf^-, Br^-, I^-, Al[OC(CH_3)_3]_4^-$

EP 2428501A1 describes a similar method for the synthesis of diaryliodonium salt mixtures with long alkyl chains and obtaining them as bromide and iodide salts only by adding aqueous KBr- or respectively KI-solutions to the reaction mixture. The precipitated products were then recrystallized. Such initiators with bromides and iodides are known to exhibit higher crystallization tendency and less solubility, making them less useful and technically applicable as photoinitiators, in particular in offset printing plates. Direct isolation of diaryliodonium mixtures in the form of hydrogen sulfate salts has proven to be surprisingly advantageous by giving highly pure products as crystalline materials, including all given subsequent products with exchanged counteranion. Even mixtures of > 6 different components could be transferred into solid compositions from oily intermediates.

**Use of diaryliodonium salt mixtures according to the invention**

The topic of the invention is also the use of the diaryliodonium salt mixtures as initiators for polymerization procedures in chemical and material applications, particularly for polymerization processes based on radical and/or cationic reaction mechanisms. This includes processes of curing, hardening and finishing, for instance for printing plate layers, 3D printing materials, coatings, varnishes, glues, adhesives, sealants, abrasives, pastes, foils, precursors for holographic materials, stereolithography, optical components and electronic components.

Preferred use of the compounds according to the invention is as photoinitiators for methods of photopolymerization, as known for the skilled person (i.e. Baumann et al.: Imaging Technology, 3. Imaging in Graphic Arts; In: Ullmann's Encyclopedia of Industrial Chemistry, 2015 Wiley-VCH Verlag GmbH & Co; Dufour, P. Radiation Curing in Polymer Science and Technology-Vol I: Fundamentals and Methods; Fouassier, J.P., Rabek, J.F., Eds.; Elsevier Science Publishers: London, UK; New York, NY, USA, 1993; pp. 1-28; Sangermano et al.; Polymers 2018, 10, 136). Photopolymerization can be initiated at different wavelengths of the spectral range from UV to near-IR, optionally supported combining the photoinitiators by sensitizing dyes. Photocuring in 3D printing technologies is described by Quan H. et al.; Bioactive Materials 5 (2020) 110-115. Applications for hologram storage and waveguide formation are described by Malallah R. et al. Polymers 2017, 9, 337.

Within this scope, preferred use of the compounds as photoinitiators encompass those compounds of formula (I) - (X), wherein the anion $Z^-$ influences the mechanistic pathway of photopolymerization. For radical photopolymerization, the anion $Z^-$ is preferably selected from the group comprising tetraphenylborate, triphenyl-monoalkylborate. Accordingly, more preferred is the use of diaryliodonium salt mixtures comprising the anions tetraphenylborate or triphenyl-monoalkylborate as photoinitiators for photopolymerization based on radicalic mechanisms. Most preferred is the respective use of compounds 1, 2, 7, 8, 13, 14, 19, 20, 25, 26, 31, 32, 37, 38, 43, 44, 49, 50, 55, 56.

Preferred anions efficient for cationic photopolymerization are selected from the group comprising hexafluorophosphate, tetrakis(perfluoro-tert-butoxy)aluminate $[((CF_3)_3C\text{-}O)_4Al^-)]$ and tetrakis(penta-fluorophenyl)borate. Accordingly, more preferred is the use of diaryliodonium salt mixtures comprising these anions as photoinitiators for cationic photopolymerization. Most preferred is the respective use of compounds 3, 4, 9, 10, 15, 16, 19, 21, 22, 27, 28, 33, 34, 39, 40, 45, 46, 51, 52, 57, 58.

Preferred anions to achieve increased thermal stability are selected from the group comprising tris(pentafluoroethyl)trifluorophosphate and bis(trifluoromethansulfonyl)imide ($NTf_2$; $(CF_3SO_2)_2N^-$).

**Application in CTP**

As used herein to define various components of the infrared radiation-sensitive imageable layer and formulation, unless otherwise indicated, the singular forms "a," "an," and "the," are intended to include one or more of the components (that is, including plurality referents). Each term that is not explicitly defined in the present application is to be understood to have a meaning that is commonly accepted by those skilled in the art. If the construction of a term would render it meaningless or essentially meaningless in its context, the term definition should be given its conventional meaning.

The use of numerical values in the various ranges specified herein, unless otherwise expressly indicated, are considered to be approximations as though the minimum and maximum values within the stated ranges were both preceded by the word "about." In this manner, slight variations above and below the stated ranges can be used to achieve substantially the same results as the values within the ranges. In addition, the disclosure of these ranges is intended as a continuous range including every value between the minimum and maximum values. Unless the context indicates otherwise, when used herein, the terms "negative-working, infrared radiation-sensitive lithographic printing plate precursor," "precursor," and "lithographic printing plate precursor" are meant to be equivalent references to embodiments of the present invention. The term "support" is used herein to refer to an aluminum-containing material (web, sheet, foil, or other form) that can be then treated or coated to prepare a "substrate" that refers to a hydrophilic article having a hydrophilic surface upon which various layers, including the infrared radiation-sensitive imageable layer, and optional hydrophilic overcoat are coated. As used herein, the term "infrared radiation absorber" refers to compounds or materials that are sensitive to wavelengths of infrared radiation. As used herein, the term "infrared" refers to radiation having a $\lambda$max of at least 750 nm and higher. In most instances, the term "infrared" is used to refer to the "near-infrared" region of the electromagnetic spectrum that is defined herein to be at least 750 nm and up to and including 1400 nm.

For clarification of definitions for any terms relating to polymers, reference should be made to "Glossary of Basic Terms in Polymer Science" as published by the International Union of Pure and Applied Chemistry ("IUPAC"), Pure Appl. Chem. 68, 2287-2311 (1996). However, any definitions explicitly set forth herein should be regarded as controlling.

As used herein, the term "polymer" is used to describe compounds with relatively large molecular weights formed by linking together many small reacted monomers. As the polymer chain grows, it folds back on itself in a random fashion to form coiled structures. With the choice of solvents, a polymer can become insoluble as the chain length grows and become polymeric particles dispersed in the solvent medium. These particle dispersions can be very stable and useful in infrared radiation-sensitive imageable layers described for use in the present invention. In this invention, unless indicated otherwise, the term "polymer" refers to a non-crosslinked material. Thus, crosslinked polymeric particles differ from the non-crosslinked polymeric particles in that the latter can be dissolved in certain organic solvents of good solvating property whereas the crosslinked polymeric particles may swell but do not dissolve in the organic solvent because the polymer chains are connected by strong covalent bonds. The term "copolymer" refers to polymers composed of two or more different repeating or recurring units that are arranged along the polymer backbone.

The term "backbone" refers to the chain of atoms in a polymer to which a plurality of pendant groups can be attached. An example of such a backbone is an "all carbon" backbone obtained from the polymerization of one or more ethylenically unsaturated polymerizable monomers.

The term "arranged randomly" means that blocks of recurring units are not intentionally incorporated into the polymeric binders, but that recurring units are incorporated into the backbone in a random fashion using known polymerization procedures that do not encourage the formation of block copolymers. Recurring units in polymeric binders described herein are generally derived from the corresponding ethylenically unsaturated polymerizable monomers used in a polymerization process, which ethylenically unsaturated polymerizable monomers can be obtained from various commercial sources or prepared using known chemical synthetic methods.

As used herein, the term "ethylenically unsaturated polymerizable monomer" refers to a compound comprising one or more ethylenically unsaturated (-C=C-) bonds that are polymerizable using free radical or acid-catalyzed polymerization reactions and conditions. It is not meant to refer to chemical compounds that have only unsaturated -C=C- bonds that are not polymerizable under these conditions.

Unless otherwise indicated, the term "weight %" refers to the amount of a component or material based on the total solids of a composition, formulation, or layer. Unless otherwise indicated, the percentages can be the same for either a dry layer or the total solids of the formulation or composition.

As used herein, the term "layer" or "coating" can consist of one disposed or applied layer or a combination of several sequentially disposed or applied layers. As the layer is considered infrared radiation-sensitive and negative-working, it is both sensitive to infrared radiation as described above and negative-working in the formation of lithographic printing plates.

The present invention is useful for preparing lithographic printing plates by imagewise exposing and processing the exposed precursor off-press using a suitable developer or on a suitable printing press using a lithographic printing ink, a fountain solution, or both a lithographic printing ink and a fountain solution as described below. The lithographic printing plate precursors of the present invention are prepared with the structure and components described as follows.

The substrate that is present in the precursors generally has a hydrophilic surface, or at least a surface that is more hydrophilic than the applied infrared radiation-sensitive imageable layer on the imaging side of the substrate. The substrate comprises a support that can be composed of any material that is conventionally used to prepare lithographic printing plate precursors.

One useful substrate is composed of an aluminum support that can be treated using techniques known in the art, including roughening of some type by physical (mechanical) graining, electrochemical graining, or chemical graining, usually followed by anodizing. Anodizing is typically done using phosphoric or sulfuric acid and conventional procedures.

Anodized aluminum support can be treated further to seal the oxide pores and to hydrophilize its surface using known post-anodic treatment (PAT) processes, such as treatments in aqueous solutions of poly(vinyl phosphonic acid) (PVPA), vinyl phosphonic acid copolymers, poly[(meth)acrylic acid], or acrylic acid copolymers, mixtures of phosphate and fluoride salts, or sodium silicate. Useful treatment processes include dipping with rinsing, dipping without rinsing, and various coating techniques such as extrusion coating. The thickness of a substrate can be varied but should be sufficient to sustain the wear from printing and thin enough to wrap around a printing form. Useful embodiments include a treated aluminum foil having a thickness of at least 100 μm and up to and including 700 μm. The backside (non-imaging side) of the substrate can be coated with antistatic agents, a slipping layer, or a matte layer to improve handling and "feel" of the precursor.

Infrared Radiation-Sensitive Imageable Layer: The precursors of the present invention can be formed by suitable application of a negative-working infrared radiation-sensitive composition as described below to a suitable substrate (as described above) to form an infrared radiation- sensitive imageable layer that is negative-working on that substrate. In general, the infrared radiation-sensitive composition (and resulting infrared radiation-sensitive imageable layer) comprises one or more free radically polymerizable compounds, one or more infrared radiation absorbers, an initiator composition that provides free radicals upon exposure to imaging infrared radiation, and optionally a polymeric binder. There is generally only a single infrared radiation-sensitive imageable layer in the precursor. It is generally the outermost layer in the precursor, but in some embodiments, there can be an outermost water-soluble hydrophilic overcoat (also known as a topcoat or oxygen barrier layer) disposed over the one or more infrared radiation-sensitive imageable layers.

The infrared radiation-sensitive imageable layers provided in precursors preferably comprises one or more polymeric binders that can be selected from a number of materials known in the art. For example, some useful primary polymeric binders comprise recurring units having side chains comprising polyalkylene oxide segments such as those described in U.S. Patent 6,899,994 (Huang et al.). Other useful polymeric binders comprise two or more types of recurring units having different side chains comprising polyalkylene oxide segments as described in Japanese Patent Publication 2015-202586 (Kamiya et al.). Some of such polymeric binders can further comprise recurring units having pendant cyano groups as those described in U.S. Patent 7,261,998 (Hayashi et al.).

Some useful polymeric binders are present in particulate form, that is, in the form of discrete particles (non-agglomerated particles). Such discrete particles can have an average particle size of at least 10 nm and up to and including 1500 nm, or typically of at least 80 nm and up to and including 600 nm, and that are generally distributed uniformly within the infrared radiation-sensitive imageable layer. Average particle size can be determined by various known methods including measuring the particles in electron scanning microscope images and averaging a set number of measurements. The polymeric binders also can have a backbone comprising multiple (at least two) urethane moieties as well as pendant groups comprising the polyalkylenes oxide segments. Useful polymeric binders also include those

that comprise polymerizable groups such as acrylate ester group, methacrylate ester group, vinyl aryl group and allyl group and those that comprise alkali soluble groups such as carboxylic acid. Some of these useful primary binders are described in U.S. Patent Application Publication 2015/0099229 (Simpson et al.) and U.S. Patent 6,916,595 (Fujimaki et al.).

Polymeric binders generally have a weight average molecular weight ($M_n$) of at least 2,000 and up to and including 500,000, or at least 20,000 and up to and including 300,000, as determined by Gel Permeation Chromatography (polystyrene standard). Useful polymeric binders can be obtained from various commercial sources or they can be prepared using known procedures and starting materials, as described for example in publications described above. The total polymeric binders are generally present in the infrared radiation-sensitive imageable layer in an amount of at least 10 weight % and up to and including 70 weight %, or more likely in an amount of at least 20 weight % and up to and including 50 weight %, based on the total dry weight of the infrared radiation-sensitive imageable layer.

The infrared radiation-sensitive composition (and infrared radiation-sensitive imageable layer) comprises one or more free radically polymerizable compounds, each of which contains one or more free radically polymerizable groups (and two or more of such groups in some embodiments) that can be polymerized using free radical initiation. In some embodiments, the infrared radiation-sensitive imageable layer comprises two or more free radically polymerizable components having different numbers of free radically polymerizable groups in each molecule.

Useful free radically polymerizable components can contain one or more free radical polymerizable monomers or oligomers having one or more addition polymerizable ethylenically unsaturated groups (for example, two or more of such groups). Similarly, crosslinkable polymers having such free radically polymerizable groups can also be used. Oligomers or prepolymers, such as urethane acrylates and methacrylates, epoxide acrylates and methacrylates, polyester acrylates and methacrylates, polyether acrylates and methacrylates, and unsaturated polyester resins can be used. In some embodiments, the free radically polymerizable component comprises carboxyl groups.

Free radically polymerizable components include urea urethane (meth)acrylates or urethane (meth)acrylates having multiple (two or more) polymerizable groups. Mixtures of such compounds can be used, each compound having two or more unsaturated polymerizable groups, and some of the compounds having three, four, or more unsaturated polymerizable groups. For example, a free radically polymerizable component can be prepared by reacting DESMODUR® N100 aliphatic polyisocyanate resin based on hexamethylene diisocyanate (Bayer Corp., Milford, Conn.) with hydroxyethyl acrylate and pentaerythritol triacrylate. Useful free radically polymerizable compounds include NK Ester A-DPH (dipentaerythritol hexaacrylate) that is available from Kowa American, and Sartomer 399 (dipentaerythritol pentaacrylate), Sartomer 355 (ditrimethylolpropane tetraacrylate), Sartomer 295 (pentaerythritol tetraacrylate), and Sartomer 415 [ethoxylated (20)trimethylolpropane triacrylate] that are available from Sartomer Company, Inc.

Numerous other free radically polymerizable components are known in the art and are described in considerable literature including Photoreactive Polymers: The Science and Technology of Resists, A Reiser, Wiley, New York, 1989, pp. 102-177, by B.M. Monroe in Radiation Curing: Science and Technology, S.P. Pappas, Ed., Plenum, New York, 1992, pp. 399-440, and in "Polymer Imaging" by A.B. Cohen and P. Walker, in Imaging Processes and Material, J.M. Sturge et al. (Eds.), Van Nostrand Reinhold, New York, 1989, pp. 226-262. For example, useful free radically polymerizable components are also described in EP1182033A1 (Fujimaki et al.), beginning with paragraph [0170], and in U.S Patents 6,309,792 (Hauck et al.), 6,569,603 (Furukawa), and 6,893,797 (Munnelly et al.). Other useful free radically polymerizable components include those described in U.S. Patent Application Publication 2009/0142695 (Baumann et al.), which radically polymerizable components include 1H-tetrazole groups.

The one or more free radically polymerizable compounds are generally present in an infrared radiation-sensitive imageable layer in an amount of at least 10 weight % and up to and including 70 weight %, or typically of at least 20 weight % and up to and including 50 weight %, all based on the total solids in the noted layer.

In addition, the infrared radiation-sensitive composition (and imageable layer) also comprises one or more infrared radiation absorbers to provide desired radiation sensitivity. The total amount of one or more infrared radiation absorbers is at least 0.5 weight % and up to and including 30 weight %, or typically of at least 1 weight % and up to and including 15 weight %, based on the infrared radiation-sensitive composition (or imageable layer) total solids.

Some useful infrared radiation absorbers are sensitive to both infrared radiation (typically of at least 700 nm and up to and including 1400 nm) and visible radiation (typically of at least 450 nm and up to and including 700 nm). Useful infrared radiation absorbers are described in U.S. Patent No. 7,429,445 (Munnelly et al.).

In many embodiments of this invention, the present invention comprises one or more infrared radiation absorbers that are sensitive only to near-infrared or infrared radiation having a wavelength of at least 750 nm. Such useful infrared radiation absorbers include but are not limited to, azo dyes, squarilium dyes, croconate dyes, triarylamine dyes, thioazolium dyes, indolium dyes, oxonol dyes, oxazolium dyes, cyanine dyes, merocyanine dyes, phthalocyanine dyes, indocyanine dyes, indotricarbocyanine dyes, oxatricarbocyanine dyes, thiocyanine dyes, thiatricarbocyanine dyes, cryptocyanine dyes, naphthalocyanine dyes, polyaniline dyes, polypyrrole dyes, polythiophene dyes, chalcogenopyryloarylidene and bi(chalcogenopyrylo) polymethine dyes, oxyindolizine dyes, pyrylium dyes, pyrazoline azo dyes, oxazine dyes, naphthoquinone dyes, anthraquinone dyes, quinoneimine dyes, methine dyes, arylmethine dyes, squarine dyes,

oxazole dyes, croconine dyes, porphyrin dyes, and any substituted or ionic form of the preceding dye classes. Suitable dyes are also described in U.S. Patents 5,208,135 (Patel et al.), 6,153,356 (Urano et al.), 6,264,920 (Achilefu et al.), 6,309,792 (Hauck et al.), 6,569,603 (Furukawa), 6,787,281 (Tao et al.), 7,018,775 (Tao), 7,135,271 (Kawaushi et al.), WO 2004/101280 (Munnelly et al.), and EP1182033A1 (noted above). In some embodiments, it is desirable that at least one infrared radiation absorber in the infrared radiation-sensitive imageable layer be a cyanine dye comprising a tetraarylborate anion such as a tetraphenylborate anion.

The infrared radiation-sensitive imageable layer essentially comprises an initiator composition according to invention as described above. The essential compounds of the diaryl iodonium salt mixture are individually and collectively capable of generating free radicals sufficient to initiate polymerization of the various free radically polymerizable compounds described above upon exposure to imaging infrared radiation. Thus, the initiator composition is generally responsive, for example, to electromagnetic radiation of at least 750 nm and up to and including 1400 nm or at least 750 nm and up to and including 1250 nm. The initiator composition can be used for any of the noted infrared radiation exposures or for multiple infrared radiation exposures.

The initiator composition is present in the infrared radiation-sensitive imageable layer sufficient to provide a total (cumulative) amount of compounds of formula (I) to (X) of at least 3 weight % and up to and including 30 weight %, or typically of at least 5 weight % and up to and including 18 weight %, or even at least 7 weight % and up to and including 15 weight %, all based on the total solids in the infrared radiation-sensitive imageable layer.

Additional additives to the infrared radiation-sensitive imageable layer can include dye precursors and color developers as are known in the art. Useful dye precursors include but are not limited to, phthalide and fluoran leuco dyes having a lactone skeleton with an acid dissociation property, such as those described in U.S. Patent 6,858,374 (Yanaka).

The infrared radiation-sensitive imageable layer can include crosslinked polymer particles having an average particle size of at least 3 μm and up to and including 20 μm as described for example in U.S. Serial No. 14/642,876 (filed March 10, 2015 by Hayakawa et al.) and in U.S. Patents 8,383,319 (Huang et al.) and 8,105,751 (Endo et al).

The infrared radiation-sensitive imageable layer can also include a variety of other optional compounds including but not limited to, dispersing agents, humectants, biocides, plasticizers, surfactants for coatability or other properties, viscosity builders, pH adjusters, drying agents, defoamers, preservatives, antioxidants, development aids, rheology modifiers, adhesion promoters, or combinations thereof, or any other addenda commonly used in the lithographic art, in conventional amounts.

Hydrophilic Overcoat: While in some embodiments of the present invention, the infrared radiation-sensitive imageable layer is the outermost layer with no layers disposed thereon, it is possible that the precursors could be designed with a hydrophilic overcoat (or oxygen-barrier layer or topcoat) disposed directly on the infrared radiation-sensitive imageable layer (no intermediate layers between these two layers). Such precursors could be developed on-press as well as off-press using any suitable developer as described below. When present, this hydrophilic overcoat is generally the outermost layer and thus, when stacked with other precursors, the hydrophilic overcoat of one precursor would be in contact with the backside of the substrate of the precursor immediately above it.

Such hydrophilic overcoats can comprise one or more film-forming water-soluble polymeric binders in an amount of at least 60 weight % and up to and including 98 weight %, based on the total dry weight of the hydrophilic overcoat. Such film-forming water-soluble (or hydrophilic) polymeric binders can include a modified or unmodified poly(vinyl alcohol) having a saponification degree of at least 30%, or a degree of at least 75%, or a degree of at least 90%, and a degree of up to and including 99.9%.

Further, one or more acid-modified poly(vinyl alcohol)s can be used as film-forming water-soluble (or hydrophilic) polymeric binders in the hydrophilic overcoat. For example, at least one modified poly(vinyl alcohol) can be modified with an acid group selected from the group consisting of carboxylic acid, sulfonic acid, sulfuric acid ester, phosphonic acid, and phosphoric acid ester groups. Examples of such materials include but are not limited to, sulfonic acid-modified poly(vinyl alcohol), carboxylic acid-modified poly(vinyl alcohol), and quaternary ammonium salt-modified poly(vinyl alcohol), glycol-modified poly(vinyl alcohol), or combinations thereof.

The optional hydrophilic overcoat can also include crosslinked polymer particles having an average particle size of at least 3 μm and up to and including 20 μm as described for example in U.S. Serial No. 14/642,876 (filed March 10, 2015 by Hayakawa et al.) and in U.S. Patents 8,383,319 (Huang et al.) 8,105,751 (Endo et al).

When present, the hydrophilic overcoat is provided at a dry coating coverage of at least 0.1 $g/m^2$ and up to but less than 4 $g/m^2$, and typically at a dry coating coverage of at least 0.15 $g/m^2$ and up to and including 2.5 $g/m^2$. In some embodiments, the dry coating coverage is as low as 0.1 $g/m^2$ and up to and including 1.5 $g/m^2$ or at least 0.1 $g/m^2$ and up to and including 0.9 $g/m^2$, such that the hydrophilic overcoat layer is relatively thin for easy removal during off-press development or on-press development.

The hydrophilic overcoat can optionally comprise organic wax particles dispersed, generally uniformly, within the one or more film-forming water-soluble (or hydrophilic) polymeric binders as described for example in U.S. Patent Application Publication 2013/0323643 (Balbinot et al.).

Negative-working Infrared Radiation-sensitive Lithographic Printing Plate Precursors: The negative-working infrared radiation-sensitive compositions described above can be applied to a substrate as a solution or dispersion in a coating liquid using any suitable equipment and procedure, such as spin coating, knife coating, gravure coating, die coating, slot coating, bar coating, wire rod coating, roller coating, or extrusion hopper coating. They can also be applied by spraying onto a suitable support. Typically, the negative-working infrared radiation-sensitive composition is applied and dried to form an infrared radiation-sensitive imageable layer.

Such manufacturing methods can include mixing the various components needed for the imaging chemistry in a suitable organic solvent or mixtures thereof [such as methyl ethyl ketone (2-butanone), methanol, ethanol, 1-methoxy-2-propanol, iso-propyl alcohol, acetone, $\gamma$-butyrolactone, n-propanol, tetrahydrofuran, and others readily known in the art, as well as mixtures thereof], applying the resulting solution to a substrate, and removing the solvent(s) by evaporation under suitable drying conditions. After proper drying, the dry coating coverage of the infrared radiation-sensitive imageable layer is generally at least 0.1 $g/m^2$ and up to and including 4 $g/m^2$ or at least 0.4 $g/m^2$ and up to and including 1.8 $g/m^2$.

Distinct non-imageable layers can also be present under the infrared radiation-sensitive imageable layer and disposed directly on the hydrophilic substrate to enhance developability or to act as thermal insulating layers. However, unless a hydrophilic overcoat layer is present, there are no layers disposed over the infrared radiation-sensitive imageable layer.

As noted above, in some embodiments, a suitable aqueous-based hydrophilic overcoat formulation (as described above) can be applied to the dried infrared radiation-sensitive imageable layer in a suitable manner, and then dried in a suitable manner.

Imaging (Exposing) Conditions: During use, a negative-working infrared radiation-sensitive lithographic printing plate precursor of this invention can be exposed to a suitable source of exposing radiation depending upon the infrared radiation absorber present in the infrared radiation-sensitive imageable layer to provide specific sensitivity that is at a wavelength of at least 750 nm and up to and including 1400 nm, or of at least 800 nm and up to and including 1250 nm using an appropriate energy source. For example, imaging can be carried out using imaging or exposing radiation from an infrared radiation-generating laser (or array of such lasers). Imaging also can be carried out using imaging radiation at multiple wavelengths at the same time if desired. The laser used to expose the precursor is usually a diode laser, because of the reliability and low maintenance of diode laser systems, but other lasers such as gas or solid-state lasers can also be used. The combination of power, intensity and exposure time for infrared radiation laser imaging would be readily apparent to one skilled in the art.

The imaging apparatus can be configured as a flatbed recorder or as a drum recorder, with the negative-working infrared radiation-sensitive lithographic printing plate precursor mounted to the interior or exterior cylindrical surface of the drum. An example of useful imaging apparatus are available as models of KODAK® Trendsetter platesetters (Eastman Kodak Company) that contain laser diodes that emit near infrared radiation at a wavelength of about 830 nm. Other suitable imaging apparatus includes the Screen PlateRite® 4300 series or 8600 series platesetter (available from Screen USA, Chicago, IL) that operates at a wavelength of 810 nm.

Imaging with infrared radiation can be carried out generally at imaging energies of at least 30 $mJ/cm^2$ and up to and including 500 $mJ/cm^2$ and typically at least 50 $mJ/cm^2$ and up to and including 300 $mJ/cm^2$ depending upon the sensitivity of the infrared radiation-sensitive imageable layer.

Processing (Development) and Printing: After imagewise exposing, the exposed negative-working infrared radiation-sensitive lithographic printing plate precursors having exposed regions and non-exposed regions in the infrared radiation-sensitive imageable layer are processed in a suitable manner to remove the non-exposed regions (and any hydrophilic overcoat over such regions).

As noted above, processing can be carried out off-press using any suitable developer in one or more successive applications (treatments or developing steps) of the same or different processing solution. Such one or more successive processing treatments can be carried out with exposed precursors for a time sufficient to remove the non-exposed regions of the infrared radiation-sensitive imageable layer to reveal the hydrophilic surface of the substrate, but not long enough to remove significant amounts of the exposed regions that have been hardened in the same layer. During lithographic printing, the revealed hydrophilic substrate surface repels inks while the remaining exposed regions accept lithographic printing ink.

Prior to such off-press processing, the exposed precursors can be subjected to a "pre-heating" process to further harden the exposed regions in the infrared radiation-sensitive imageable layer. Such optional pre-heating can be carried out using any known process and equipment generally at a temperature of at least 60°C and up to and including 180°C.

Following this optional pre-heating, or in place of the pre-heating, the exposed precursor can be washed (rinsed) to remove any hydrophilic overcoat that is present. Such optional washing (or rinsing) can be carried out using any suitable aqueous solution (such as water or an aqueous solution of a surfactant) at a suitable temperature and for a suitable time that would be readily apparent to one skilled in the art.

Useful developers can be ordinary water or can be formulated. The formulated developers can comprise one

or more components selected from surfactants, organic solvents, alkali agents, and surface protective agents.

Useful organic solvents include the reaction products of phenol with ethylene oxide and propylene oxide [such as ethylene glycol phenyl ether (phenoxyethanol)], benzyl alcohol, esters of ethylene glycol and of propylene glycol with acids having 6 or less carbon atoms, and ethers of ethylene glycol, diethylene glycol, and of propylene glycol with alkyl groups having 6 or less carbon atoms, such as 2-ethylethanol and 2-butoxyethanol.

In some instances, an aqueous processing solution can be used off-press to both develop the imaged precursor by removing the non-exposed regions and also to provide a protective layer or coating over the entire imaged and developed (processed) precursor printing surface. In this embodiment, the aqueous solution behaves somewhat like a gum that is capable of protecting (or "gumming") the lithographic image on the printing plate against contamination or damage (for example, from oxidation, fingerprints, dust, or scratches).

After the described off-press processing and optional drying, the resulting lithographic printing plate can be mounted onto a printing press without any contact with additional solutions or liquids. It is optional to further bake the lithographic printing plate with or without blanket or floodwise exposure to UV or visible radiation.

After off-press developing, printing can be carried out by putting the exposed and processed lithographic printing plate on a suitable printing press. Printing can be carried out by applying a lithographic printing ink and fountain solution to the printing surface of the lithographic printing plate in a suitable manner. The fountain solution is taken up by the surface of the hydrophilic substrate revealed by the exposing and processing steps, and the lithographic ink is taken up by the remaining (exposed) regions of the imageable layer. The lithographic ink is then transferred to a suitable receiving material (such as cloth, paper, metal, glass, or plastic) to provide a desired impression of the image thereon. If desired, an intermediate "blanket" roller can be used to transfer the lithographic ink from the lithographic printing plate to the receiving material (for example, sheets of paper).

On-Press Development and Printing: Alternatively, an imaged negative-working infrared radiation-sensitive lithographic printing plate precursor is mounted onto a printing press and the printing operation is begun. The non-exposed regions in the infrared radiation-sensitive imageable layer are removed by a suitable fountain solution, lithographic printing ink, or a combination of both, when the initial printed impressions are made. Typical ingredients of aqueous fountain solutions include pH buffers, desensitizing agents, surfactants and wetting agents, humectants, low boiling solvents, biocides, antifoaming agents, and sequestering agents. A representative example of a fountain solution is Varn Litho Etch 142W + Varn PAR (alcohol sub) (available from Varn International, Addison, IL).

In a typical printing press startup with a sheet-fed printing machine, the dampening roller is engaged first and supplies fountain solution to the mounted imaged precursor to swell the exposed infrared radiation-sensitive imageable layer at least in the non-exposed regions. After a few revolutions, the inking rollers are engaged, and they supply lithographic printing ink(s) to cover the entire printing surface of the lithographic printing plates. Typically, within 5 to 20 revolutions after the inking roller engagement, printing sheets are supplied to remove the non-exposed regions of the infrared radiation-sensitive imageable layer as well as materials on a blanket cylinder if present, using the formed ink-fountain emulsion.

**Applications in cationic polymerization**

According to the present invention, cationic polymerization is mainly understood as ring-opening polymerization, as known to the skilled person. Polymerizable monomers or oligomers are usually epoxides (oxiranes), vinylethers, thiiranes (episulfides), oxetanes, aziridines, lactames, lactones, lactids, glycolids, tetrahydrofuran, organopolysiloxanes containing epoxy groups, organopolysiloxanes containing alkenyloxy groups. Highest relevance in the application have monomeric or oligomeric epoxides. Materials and methods are known to the skilled person, as published by Sangermano M.; Macromolecular Materials and Engineering 2014, 299 (7), 775-793.

In the field of epoxy-based curing, the diaryliodonium salt mixtures are applied in combination with epoxy resins of the types established for diverse industrial applications, such as bisphenol A types. These include several trades, such as SU-8 novolack, EPON, EPALLOY, DER, ARALDITE, ECN cresol analogs. The application is meant to be conducted together with usual reactive additives, thinners, solvents, fillers, initiators, stabilizers, modifiers, as needed for the respective material to be obtained.

Beside the preferred anion hexafluorophosphate and tetrakis(perfluoro-tert-butoxy)aluminate [$((CF_3)_3C\text{-}O)_4Al^-$)] being efficient for cationic photopolymerization, there are several other anions described as possible structures to enable photoacid generation; see I . Raabe, A. Reisinger und I. Krossing, "Efficient syntheses of Li[Al(ORF)], Ag[Al(ORF)] (RF = C(CF3)3, C(H)(CF 3)2, C(CH3)(CF 3)2)) and [H(OEt 2)2]+ [Al(OC(CF 3)3)] " , H. W. Roesky und Dietmar K. Kennepohl (Edit.), Experiments in Green and Sustainable Chemistry, Wiley VCF, S. 13 1-144 (2009). Structurally defined iodonium salts with aluminate anion are described in WO 2017/035552A.

## Description of Figures

**Figure 1:** Possible iodonium cations formed as components of a mixture, when 3 different starting materials (n = 3) are used. As example, not limiting the scope of the invention, starting materials depicted are alkylaryl compounds t-butylbenzene, i-propylbenzene (Cumene), and toluene. The mixture contains para/para-structures (4-substitution), ortho/para-structures (2- and 4-substitution) and ortho/ortho-structures (2-substitution).

**Figure 2:**
Synthesis of diaryliodonium salt mixtures from two starting components (t-butylbenzene, i-propylbenzene) yielding the product mix as hydrogen sulfate salts (possible o/p- and o/o-isomers not depicted; theoretical number of all possible isomers is 10).

**Figure 3:**
Synthesis of diaryliodonium salt mixtures from three starting components (t-butylbenzene, i-propylbenzene, toluene) yielding the product mix as hydrogen sulfate salts (possible o/p- and o/o-isomers not depicted; theoretical number of all possible isomers is 21).

**Figure 4:**
Synthesis of diaryliodonium salt mixtures from three starting components (t-butylbenzene, i-propylbenzene, anisole) yielding the product mix as hydrogen sulfate salts (possible o/p- and o/o-isomers not depicted; theoretical number of all possible isomers is 21).

**Figure 5:**
Synthesis of diaryliodonium salt mixtures from four starting components (t-butylbenzene, i-propylbenzene, ethylbenzene, toluene) yielding the product mix as hydrogen sulfate salts (possible o/p- and o/o-isomers not depicted; theoretical number of all possible isomers is 56).

**Figure 6:**
H-NMR spectrum and HPLC chromatogram of diaryliodonium mixture from starting components t-butylbenzene, i-propylbenzene with hexafluorophosphate anion (compound **3).** Figure 6A shows H-NMR (DMSO-$d_6$, 700 MHz) and Figure 6B shows HPLC chromatogram (RP-C18 Purospher, phosphate-buffer/acetonitrile gradient, detection 254 nm). * indicates entities with o/p or o/o-substitution

**Figure 7:**
H-NMR spectrum and HPLC chromatogram of diaryliodonium mixture from starting components t-butylbenzene, toluene. Figure 7A shows H-NMR (DMSO-$d_6$, 700 MHz) of compound **15** (hexafluorophosphate anion) and Figure 7B shows HPLC chromatogram (RP-C18 Purospher, phosphate-buffer/acetonitrile gradient, detection 254 nm) of compound **13** (tetraphenylborate anion).
* indicates entities with o/p or o/o-substitution

## Examples

The following Examples are provided to illustrate the practice of this invention and are not meant to be limiting in any manner.

**Example 1:** Synthesis of a diaryliodonium mixture based on tert-butylbenzene and *iso*-propylbenzene.

**Example 1.1:** Synthesis of diaryliodonium mixtures as hydrogen sulfates

0.31 mol (48 ml, 41.9 g) tert-butylbenzene, 0.31 mol (43.5 ml, 37.6 g) iso-propylbenzene, 1.06 mol (100 ml, 108 g) acetic anhydride and 1.75 mol (100 ml, 105 g) acetic acid were mixed in a 500 ml flask. To this solution 0.31 mol (61.9 g) $NaIO_3$ was added in small portions. The resulting suspension was cooled to 5 °C. Then, 0.68 mol (35 ml, 64.4 g) conc. $H_2SO_4$ was added dropwise while the reaction temperature must be kept below 20 °C. After adding conc. $H_2SO_4$ the cooling bath was removed, and the reaction mixture was stirred overnight. The suspension was subsequently added to a mixture of 400 g ice and 250 ml $CHCl_3$. The resulting 2-phase mixture was stirred intensely for 1 h. The organic phase was separated and neutralized via adding 500 ml of saturated $Na_2CO_3$-solution. The subsequently separated

organic phase was dried over $Na_2SO_4$. After filtration, the filtrate was added to 1.25 l $Et_2O$ and the diaryliodonium salt mixture as hydrogen sulfate precipitated in the form of white crystals. The resulting suspension was filtered while the filter cake was washed two times with 50 ml $Et_2O$ and dried at room temperature. 61.4 g of the diaryliodonium salt mixture as hydrogen sulfate with a yield of 41.2% was isolated in a white crystal form ($T_m$ = 153 - 157°C). Regarding the following anion exchange the diaryliodonium salt mixture as hydrogen sulfate was dissolved in 300 ml water and preserved as **solution A.**

According to the table below, the oxidizing agent $NaIO_3$ may be replaced by potassium iodate or sodium periodate with comparable yields.

Synthesis yields comparing different oxidizing agents according to method of example 1

| Oxidizing agent | yield [%] |
|---|---|
| **$NaIO_3$** | 41.2 |
| **$KIO_3$** | 39.5 |
| **$NaIO_4$** | 40.7 |

**Example 1.2: Exchange of counteranions**

0.31 mol (107 g) sodium tetraphenylborate was dissolved in 800 ml water **(solution B)** and filtered. Afterwards, **solution A** was added dropwise to the filtrate of **solution B.** The resulting white precipitate was filtered and washed two times with 300 ml water. After drying under reduced pressure at 60°C, 85.65 g of the target diaryliodonium salt mixture as tetraphenylborate **(compound 1)** was isolated with a yield of 90.0% ($T_m$ = 135 - 140°C). In addition to the tetraphenylborate, the diaryliodonium salt mixture was also synthesized as iodide, bromide, hexafluorophosphate (**compound 3**) and tetrakisperfluoro-tert-butoxyaluminate (**compound 4**). All yields obtained after anion exchange were at 85.0 - 97%.

**Example 2:** Synthesis of diaryliodonium salt mixture based on *tert*-butylbenzene, *iso*-propylbenzene and toluene.

0.31 mol (48 ml, 41.9 g) *tert*-butylbenzene, 0.31 mol (43.5 ml, 37.6 g) *iso*-propylbenzene, 0.313 mol (33 ml, 28.9 g) toluene, 1.06 mol (100 ml, 108 g) acetic anhydride and 1.75 mol (100 ml, 105 g) acetic acid were mixed in a 500 ml flask. Subsequently, 0.31 mol (61.9 g) $NaIO_3$ was added into small portions. Work-up and anion exchange were performed as described in Example 1. The following table shows the resulting yields after anion exchange.

Yields and melting points of diaryliodonium salt mixtures based on *tert*-butylbenzene, *iso*-propylbenzene and toluene as iodide, bromide, hexafluorophosphate, tetraphenylborate and tetrakisperfluoro-tert-butoxyaluminate

| Compound-No. | Salt solution AX | Yield Y [%] | Melting point $T_m$ [°C] |
|---|---|---|---|
| **-** | KBr | 91 | 185 - 186 |
| **-** | KI | 88 | 146 - 148 |
| **31** | $NaBPh_4$ | 87 | 120 - 125 |
| **33** | $KPF_6$ | 90 | 130 - 133 |
| **34** | $Li\{Al[OC(CF_3)_3]_4\}$ | 73 | 74 - 94 |

**Example 3:** Synthesis of diaryliodonium salt mixture based on *tert*-butylbenzene, *iso*-propylbenzene and methoxybenzene (anisole).

0.31 mol (48 ml, 41.9 g) tert-butylbenzene, 0.31 mol (43.5 ml, 37.6 g) *iso*-propylbenzene, 0.31 mol (34 ml, 33.9 g) methoxybenzene, 1.06 mol (100 ml, 108 g) acetic anhydride and 1.75 mol (100 ml, 105 g) acetic acid were mixed in a 500 ml flask. Subsequently, 0.31 mol (61.94g) $NaIO_3$ was added repeatedly by small portions. Work-up and anion exchange was performed as described in Example 1. The following table shows the resulting yields after anion exchange.

Yield and melting points of diaryliodoniumsalt mixture based on *tert*-butylbenzene, *iso*-propylbenzene and methoxybenzene (anisol) as iodide, bromide, hexafluorophosphate, tetraphenylborate and tetrakisperfluoro-tert-butoxyaluminate

| Compound-No. | Salt solution AX | Yield Y [%] | Melting point $T_m$ [°C] |
|---|---|---|---|
| **-** | KBr | 36 | 175 - 178 |
| **-** | KI | 38 | 142 - 146 |
| **55** | $NaBPh_4$ | 40 | 135 - 140 |
| **57** | $KPF_6$ | 40 | 127 - 133 |
| **58** | $Li\{Al[OC(CF_3)_3]_4\}$ | 31 | 80 - 96 |

**Example 4:** Synthesis of diaryliodonium salt mixture based on *tert*-butylbenzene, *iso*-propylbenzene, ethylbenzene and toluene.

0.31 mol (48 ml, 41.9 g) tert-butylbenzene, 0.31 mol (43.5 ml, 37.6 g) *iso*-propylbenzene, 0.31 mol (32.9 ml, 28.8 g) toluene, 0.31 mol (38.3 ml, 33.2 g) ethylbenzene, 1.06 mol (100 ml, 108 g) acetic anhydride and 1.75 mol (100 ml, 105 g) acetic acid were mixed in a 500 ml flask. 0.31 mol (62 g) $NaIO_3$ was added subsequently into the mixture in small portions. Work-up and anion exchange was performed as described in Example 1. The following table shows the resulting yields after anion exchange.

Yield and melting points of diaryliodonium salt mixture based on *tert*-butylbenzene, *iso*-propylbenzene, toluene and ethylbenzene as bromide, iodide, hexafluorophosphate, tetraphenylborate and tetrakisperfluoro-tert-butoxyaluminate

| Compound-No. | Salt solution AX | Yield Y [%] | Melting point $T_m$ [°C] |
|---|---|---|---|
| **-** | KBr | 41 | 180 - 182 |
| - | KI | 36 | 127 - 133 |
| **49** | $NaBPh_4$ | 33 | 128 - 133 |
| **51** | $KPF_6$ | 36 | 107 - 115 |
| **52** | $Li\{Al[OC(CF_3)_3]_4\}$ | 30 | 75 - 92 |

**Example 5:** Comparative synthesis of diaryliodonium salt mixture based on $C_{10}$ - $C_{13}$ alkyl substituted benzenes

Into a 4-neck flask (750 ml) equipped with stirrer, reflux condenser, dropping funnel, nitrogen inlet tube and thermometer were placed a benzene mixture (substituted with C10 - C13 alkyl units, 0.22 mol, 52.6 g), potassium iodate (0.11 mol, 23.4 g), acetic anhydride (0.5565 mol, 59.81 g) and acetic acid (55.0 g). Thereafter a mixture of concentrated sulfuric acid (0.34 mol, 32.9 g) and acetic acid (25.0 g) was added dropwise over 1 hour with the temperature of the reaction mixture not exceeding 40°C, followed by stirring for 2 hours at 40°C. Water (201.5 g) was added dropwise to the reaction mixture under cooling. Trichloromethane (200.0 g) was added and the reaction mixture stirred for 30 minutes. The resulting organic phase was separated. A solution from sodium tetraphenyl borate (0.28 mol, 94.9 g) in water (300.0 g) was added to the organic phase and stirred for 60 minutes. Thereafter the reaction mixture separated into two layers. The organic phase was washed three times with water and the organic phase concentrated under reduced pressure to dryness. The diaryliodonium tetraphenyl borate mixture was obtained as brown oil. (weight: 91 g, yield: 89%).

The diaryliodonium borate mixture based on $C_{10}$ - $C_{13}$ alkyl with tetrakis(perfluoro-tert-butoxy)aluminate as anion was obtained by anion exchange as described above.

**Example 6:** Efficacy in Negative-working, infrared radiation-sensitive lithographic printing plate

Negative-working, infrared radiation-sensitive lithographic printing plate precursors were prepared using a substrate that was composed of an aluminum sheet that had been subjected to an electrolytic roughening treatment in a hydrochloric acid solution to achieve an average roughness (Ra) of 0.4 μm. The aluminum sheet was then subjected to an anodizing treatment in an aqueous phosphoric acid solution to form 1.1 $g/m^2$ of an oxide film and then coated with a post-treatment aqueous solution of poly(acrylic acid) to give a dry thickness of 0.03 $g/m^2$.

Onto samples of this substrate, an infrared radiation-sensitive imageable layer is formed using a formulation shown in the following TABLE 1. Each formulation comprised a specific iodonium salt mixture shown in the TABLE 3 below. Each formulation was coated onto the substrate using a bar coater, dried at 50°C for 60 seconds, and then cooled

to room temperature to give a dry coating weight of 0.9 g/m$^2$ of the resulting infrared radiation-sensitive imageable layer, thus forming a negative-working, infrared radiation-sensitive lithographic printing plate precursor with each formulation.

TABLE 1 / Example 6: Formulation for the IR-sensitive imageable layer

| **Component** | **Amount (grams)** |
|---|---|
| Polymer dispersion | 1.056 |
| Hydroxypropyl methyl cellulose | 0.600 |
| Monomer 1 | 0.501 |
| Monomer 2 | 0.249 |
| IR dye 1 | 0.030 |
| Acid-Sensitive Dye Precursor 1 | 0.035 |
| Surfactant 1 | 0.069 |
| Iodonium salt mixture | acc. to TABLE 3 |
| 1-Propanol | 4.94 |
| 2-Butanone | 2.40 |
| 1-Methoxy-2-propanol | 4.22 |
| g-Butyrolactone | 0.14 |
| Water | 0.66 |

TABLE 2 / Example 6: Components of the IR-sensitive imageable layer

| | |
|---|---|
| Polymer dispersion | The polymer dispersion prepared according to Example 10 of EP 1,765,593, used as 23.5 weight % polymer in n-propanol/water at 80:20 weight ratio |
| Hydroxy propyl methyl cellulose | 5 weight % hydroxypropyl methyl cellulose polymer in water; the polymer is 30% methoxylated, 10% hydroxyl propoxylated and has a viscosity of 5 mPa sec in a 2% aqueous solution at 20°C. |
| Monomer 1 | Urethane acrylate prepared by reacting DESMODUR® N100 (from Bayer Corp., Milford, CT) with hydroxyethyl acrylate and pentaerythritol triacrylate at approximately 1:1.5:1.5 molar ratio (40 weight % in 2-butanone). |
| Monomer 2 | Ethoxylated (10 EO) Bisphenol A acrylate, 40 weight % in 2-butanone |
| IR dye 1 | 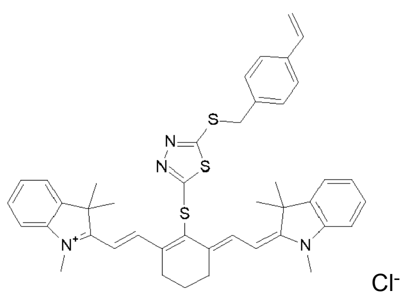 |
| Surfactant 1 | BYK® 302 from Byk Chemie, used as a 25 weight % solution in 1-methoxy-2-propanol |
| Acid-Sensitive Dye Precursor 1 | 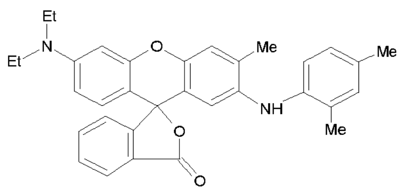 |
| comparative iodonium salt 1 | bis[4-(1,1-dimethylethyl)phenyl]-iodonium tetraphenylborate |

(continued)

| | |
|---|---|
| comparative iodonium salt 2 | bis[4-(1-methylethyl)phenyl]-iodonium tetra phenylborate |
| comparative iodonium salt 3 | bis[2,4'-(1-methylethyl)phenyl]-iodonium tetraphenylborate |
| comparative iodonium salt 4 | 4-(1-methylpropyl)phenyl-4'-(1,1-dimethylethyl)-iodonium tetraphenylborate |
| comparative iodonium salt 5 | bis[4,4'-(1-methylpropyl)phenyl]-iodonium tetraphenylborate |

TABLE 3 / Example 6: Iodonium salt mixtures

| | Iodonium salt mixture |
|---|---|
| Example 6 -1 | **Compound 1 (example 1)** |
| Comparative 1 | comparative iodonium salt 1 |
| Comparative 2 | comparative iodonium salt 2 |
| Comparative 3 | 1/1 physical mixture of Comparative iodonium salt 1 and Comparative iodonium salt 2 |
| Comparative 4 | 9/1 Mixture of Comparative iodonium salt 1 and Comparative iodonium salt 3 |
| Comparative 5 | 50/25/25 physical mixture of comparative iodonium salt 4 + comparative iodonium salt 5 + comparative iodonium salt 1 |
| Comparative 6 | product of 5 |

The negative-working, infrared radiation-sensitive lithographic printing plate precursors prepared in this manner were each imagewise exposed on a Kodak Magnus® 800 image setter to deliver a dose of 120 mJ/cm$^2$ in a solid area and thus formed exposed precursors having both exposed areas and unexposed areas in the infrared radiation-sensitive imageable layer.

Each of the negative-working, infrared radiation-sensitive lithographic printing plate precursors was evaluated in the following manner.

Crystallization Test: Each of the freshly coated negative-working infrared radiation-sensitive lithographic printing plate precursors was damaged by dragging of an ethylene propylene diene terpolymer (EPDM) rubber wheel under the pressure of 5 kg/cm$^2$ and aged 5 days at 40°C and 80% relative humidity. After this aging process, each precursor was exposed to infrared radiation using the process described above to assess the level of damage in a solid exposed region caused by crystal formation in the damaged area. The aged lithographic printing plate precursors were also examined using a scanning electron microscope (SEM) to check the amount of formed crystals. EDX analysis of the crystals revealed the presence of iodonium. The crystallization tendency was rated according to the level of damage in the solid area and the amount of crystals observed in the SEM images according to the following scale:

| | |
|---|---|
| ++ | No damage of the exposed region caused by crystal formation and no crystals in SEM images |
| + | Trace damage of the exposed region caused by crystals and very few crystals in SEM images |
| 0 | Slight damage of the exposed region caused by crystals and crystals readily in the SEM images |
| - | Moderate damage of the exposed region caused by crystals and numerous crystals in SEM images |
| -- | Severe damage and numerous crystals observed in the SEM images |

On-press Developability: Each of the lithographic printing plate precursors was exposed at 15 to 150 mJ/cm$^2$ using a Kodak Trendsetter 3244x. The exposed plate precursor was mounted on a MAN Roland Favorit 04 press machine. Fountain solution (Varn Supreme 6038) and printing ink (Gans Cyan) were supplied, and printing was performed. The on press developability was evaluated by counting the number of printed paper sheets needed to receive a clean background.

| | |
|---|---|
| + | DOP < 35 |

(continued)

| | |
|---|---|
| 0 | DOP = 35-70 |
| - | DOP > 70 |

Printing Press Life was estimated by determining the photospeed of each of the exposed lithographic printing plate precursors. The plates were exposed and developed as described above. Photospeed was measured on paper after 1000 impressions by a determination of ink density for solids exposed to different energies. The inflection point of ink density vs. exposure energy is regarded as a measure for imaging speed. The following qualitative values were given as the results of the individual experiments, with lower imaging energy desirable. The + and 0 evaluations are acceptable for this parameter.

| | |
|---|---|
| + | photospeed < 35 mJ/cm$^2$ |
| 0 | photospeed = 35 to 70 mJ/cm$^2$ |
| - | photospeed > 70 mJ/cm$^2$ |

The results of the evaluations from the three test are shown in the following TABLE 4.

TABLE 4 / Example 6

| | Photos peed | on-press developability | crystallization propensity |
|---|---|---|---|
| Example 6 -1 | 0 | 0 | ++ |
| Comparative 1 | 0 | 0 | -- |
| Comparative 2 | 0 | 0 | -- |
| Comparative 3 | 0 | 0 | 0 |
| Comparative 4 | 0 | 0 | - |
| Comparative 5 | 0 | 0 | + |
| Comparative 6 | - | 0 | ++ |

The data in TABLE 4 show that on-press developability of the printing plate precursors is not impacted by the choice of iodonium compound.

Photospeed, and hence press durability, is sufficient for all examples and comparative examples with iodonium salt initiators having low molecular weights, indicating their good photochemical properties irrespective of the complexity of their mixture, as long as the effective number of radicals formed per mass unit remains sufficient. This is not the case for comparative example 6 applying the iodonium salt mixture received in example 5, although crystallization propensity of the mixture is as good as that of the inventive mixture.

On the other hand, the crystallization propensity is high and plates tend to get damaged easily with iodonium initiators being pure components such as those in comparative examples 1 and 2. The 1/1 mixture of comparative example 3 performs better than the 9/1 mixture of comparative example 4, and comparative example 5, which has a three component mixture, shows only trace damage with the disadvantage that it required the synthesis of three components and their manual mixture, In example 6-1, no traces of damage were observed, demonstrating the usefulness of the inventive mixture as initiator for offset printing plates.

**Example 7:** Efficacy in cationic photopolymerization for curing of sol-gel coatings

A sol-gel coating solution consisting of 15% TEOS, 5% epoxy-modified silanes, 75% organic solvents, and 5% water was prepared according to protocols known (R.B. Figueira et al.; Journal of Coatings Technology and Research 2015, 12, 1-35). To 10 g of this coating solution were added 0.05 g of photoinitiator test compounds (see Table 5/example 7), followed by 1 hour of stirring at room temperature. This solution was applied to a PET foil at a wet thickness of 30 μm by a doctor blade device. This material was illuminated at 254 nm for 2 min or for 5 min (UV-Crosslinker device; UVP). The abrasion resistance (500 cycles) was determined with a standard method (taber-abraser) and the decrease in gloss was measured. Results are summarized in Table 7. Coatings with **Compound 1** showed best abrasion resistance (lowest decrease in gloss) in comparison to controls. In addition, **Compound 1** showed better solubility in the coating and layer, while the single component bis-(4-tert.-butyl-phenyl)-iodonium tetrakis(perfluoro-tert.-butoxy)aluminate lead

to visible precipitates and spots in the coat.

TABLE 5 / Example 7

| | decrease in gloss (%) 2 min UV-curing | | decrease in gloss (%) 5 min UV-curing | |
|---|---|---|---|---|
| | 100 cycles | 500 cycles | 100 cycles | 500 cycles |
| **Compound 1** 0,5% (w/w) | 19.9 | 35.5 | 17.5 | 36.8 |
| **Compound 1** 0,43 % (w/w) equimolar to Example 5 | 15.3 | 37.6 | n.d. | n.d. |
| Comparative Compound **Example 5** 0,5% (w/w) bis-($C_{10}$-$C_{13}$-alkylphenyl)-iodonium tetrakis(perfluoro-tert-butoxy) aluminate | 22.0 | 41.2 | 19.5 | 41.0 |
| Comparative Compound bis-(4-tert.-butyl-phenyl)-iodonium tetrakis (perfluoro-tert.-butoxy)aluminate 0,5% (w/w) (WO 2017/035552A) | 22.8 | 52.6 | 22.9 | 50.0 |

## Claims

1. **1.** Diaryliodonium salt mixtures, comprising at least three different, structurally defined diaryliodonium salts selected from formula (I) - (X)

| | R1 + R2 | R1 + R1 | R2 + R2 |
|---|---|---|---|
| p/p | 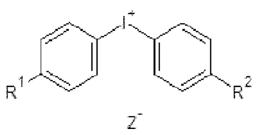 | 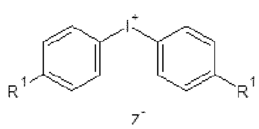 | 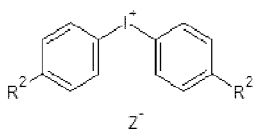 |
| | (I) | (V) | (VIII) |
| o/p | 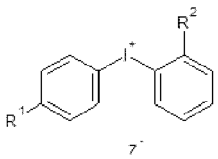 | 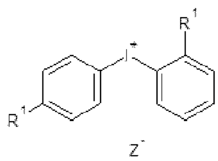 | 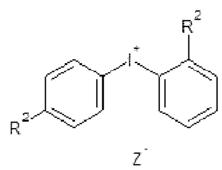 |
| | (II) | (VI) | (IX) |
| p/o | 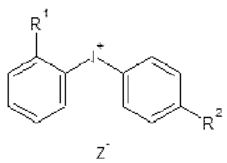 | = o/p | = o/p |
| | (III) | | |
| o/o | 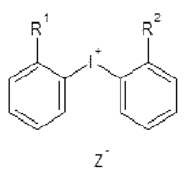 | 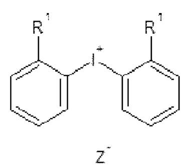 | 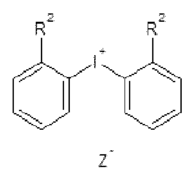 |
| | (IV) | (VII) | (X) |

wherein the components in the mixture comprise at least the structures (I), (V) and (VIII), and wherein

$R_1$ and $R_2$ are independently a linear or branched alkyl or alkoxy group of up to 7 carbon atoms, with $R_1 \neq R_2$, wherein $Z^-$ is an organic, metalloorganic or inorganic counteranion,
**characterized in that** the mixture is the statistical reaction product mixture received by reacting at least two aromatic components selected from structure A and B,

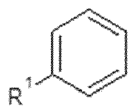 + 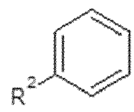 (A + B)

2. Diaryliodonium salt mixtures according to claim 1, wherein the components in the mixture comprise the structures (I), (V) and (VIII) as the minimum of three major components, and at least one additional structure selected from the formula (II), (III), (IV), (VI), (VII), (IX), (X).

3. Diaryliodonium salt mixtures according to claim 1, wherein the components in the mixture comprise the structures (I), (V) and (VIII) as the minimum of three major components, and at least two additional structures selected from the formula (II), (III), (IV), (VI), (VII), (IX), (X).

4. Diaryliodonium salt mixtures according to one of claims 1 to 3, wherein $R_1$ and $R_2$ are independently selected from the group t-butyl, s-butyl, n-butyl, i-propyl, n-propyl, ethyl, methyl, t-butyloxy, s-butyloxy, n-butyloxy, i-propyloxy, n-propyloxy, ethoxy and methoxy, and
with $Z^-$ defined according to claim 1.

5. Diaryliodonium salt mixtures according to one of claims 1 to 3, wherein $Z^-$ is an anion selected from the group chloride, bromide, iodide, perchlorate, tosylate, dodecylphenylsulfonate, hydrogensulfate, hexafluorophosphate, tris(pentafluorethyl)-trifluorphosphate, hexafluorantimonate, bis(trifluoromethane-sulfonyl)imide ($NTf_2$; $(CF_3SO_2)_2N^-$), tris(trifluoromethane-sulfonyl)methide (triflide; $CTf_3$; $(CF_3SO_2)_3C^-$), tetrafluoroborate, tetraphenylborate, triphenyl-monoalkylborate, tetrakis(penta-fluorophenyl)borate, tetrakis(di(trifluoromethyl)phenyl)borate, tetrakis(4-chlorophenyl)borate, tetrakis(perfluoro-*tert*-butoxy)aluminate [$((CF_3)_3C\text{-}O)_4Al^-$)].
with $R_1$, $R_2$ defined according to claim 1.

6. Diaryliodonium salt mixtures according to one of claims 1 to 3, wherein $Z^-$ is an anion selected from the group chloride, bromide, iodide, perchlorate, tosylate, dodecylphenylsulfonate, hydrogensulfate, hexafluorophosphate, tris(pentafluorethyl)-trifluorphosphate, hexafluorantimonate, bis(trifluoromethane-sulfonyl)imide ($NTf_2$; $(CF_3SO_2)_2N^-$), tris(trifluoromethane-sulfonyl)methide (triflide; $CTf_3$; $(CF_3SO_2)_3C^-$), tetrafluoroborate, tetraphenylborate, triphenyl-monoalkylborate, tetrakis(penta-fluorophenyl)borate, tetrakis(di(trifluoromethyl)phenyl)borate, tetrakis(4-chlorophenyl)borate, tetrakis(perfluoro-tert-butoxy)aluminate [$((CF_3)_3C\text{-}O)_4Al^-$)], and
wherein $R_1$ and $R_2$ are independently selected from the group t-butyl, s-butyl, n-butyl, i-propyl, n-propyl, ethyl, methyl, t-butyloxy, s-butyloxy, n-butyloxy, i-propyloxy, n-propyloxy, ethoxy and methoxy.

7. Diaryliodonium salt mixtures according to one of claims 1 to 3, wherein $Z^-$ is an anion selected from the group hexafluorophosphate, bis(trifluoromethane-sulfonyl)imide ($NTf_2$; $(CF_3SO_2)_2N^-$), tetrafluoroborate, tetrakis(perfluoro-tert-butoxy)aluminate [$((CF_3)_3C\text{-}O)_4Al^-$)], and
wherein $R_1$ and $R_2$ are independently selected from the group t-butyl, n-butyl, i-propyl, n-propyl, ethyl, methyl and methoxy.

8. Diaryliodonium salt mixtures according to one of claims 1 to 3, wherein $Z^-$ is tetrafluoroborate or tetrakis(perfluoro-tert-butoxy)aluminate [$((CF_3)_3C\text{-}O)_4Al^-$)], and wherein $R_1$ and $R_2$ are independently selected from the group t-butyl, n-butyl, i-propyl, n-propyl, ethyl, methyl and methoxy.

9. Method of synthesis of diaryliodonium salt mixtures according to one of claims 1 to 8 characterized that the mixture is the statistical reaction product mixture received by reacting two or more aromatic components selected from structure A and B,

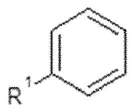 + 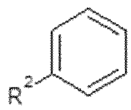 (A + B)

by applying reaction conditions with components sodium iodate, acetic anhydride, acetic acid, and sulfuric acid, obtaining the diaryliodonium salt mixtures as intermediates, and converting them into final products by anion exchange.

**10.** Method of synthesis of diaryliodonium salt mixtures according to claim 9, wherein the intermediates are obtained as hydrogen sulfate salts in the format of solid materials, which are converted further into final products by anion exchange.

**11.** Use of diaryliodonium salt mixtures according to claims 1 to 9 as photoinitiator for processes of photopolymerization.

**12.** Use of diaryliodonium salt mixtures according to claims 1 to 8 as photoinitiator for processes of photopolymerization in combination with UV-VIS or NIR-absorbing dyes acting as photosensitizers.

**13.** Use of diaryliodonium salt mixtures according to claims 1 to 8 as photoinitiators in lithographic printing plate precursors having, on a hydrophilic support, an imageable layer comprising
one or more radically polymerizable compounds,
one or more infrared radiation absorbers and
a radical initiator composition comprising a diaryliodonium salt mixture capable of forming free radicals upon exposure to NIR radiation.

**14.** Use of diaryliodonium salt mixtures according to claims 1 to 8 as photoinitiators in cationic resins precursors, containing one or more polymerizable compounds, independently selected from the group of epoxides (oxiranes), vinylethers, thiiranes (episulfides), aziridines, oxetanes, lactames, lactones, lactids, glycolids, tetrahydrofuran, organopolysiloxanes containing epoxy groups, organopolysiloxanes containing alkenyloxy groups,
together with further components, independly selected from the group of monomers, additives, thinners, solvents, fillers, co-initiators, stabilizers, modifiers or absorbers.

# Figure 1

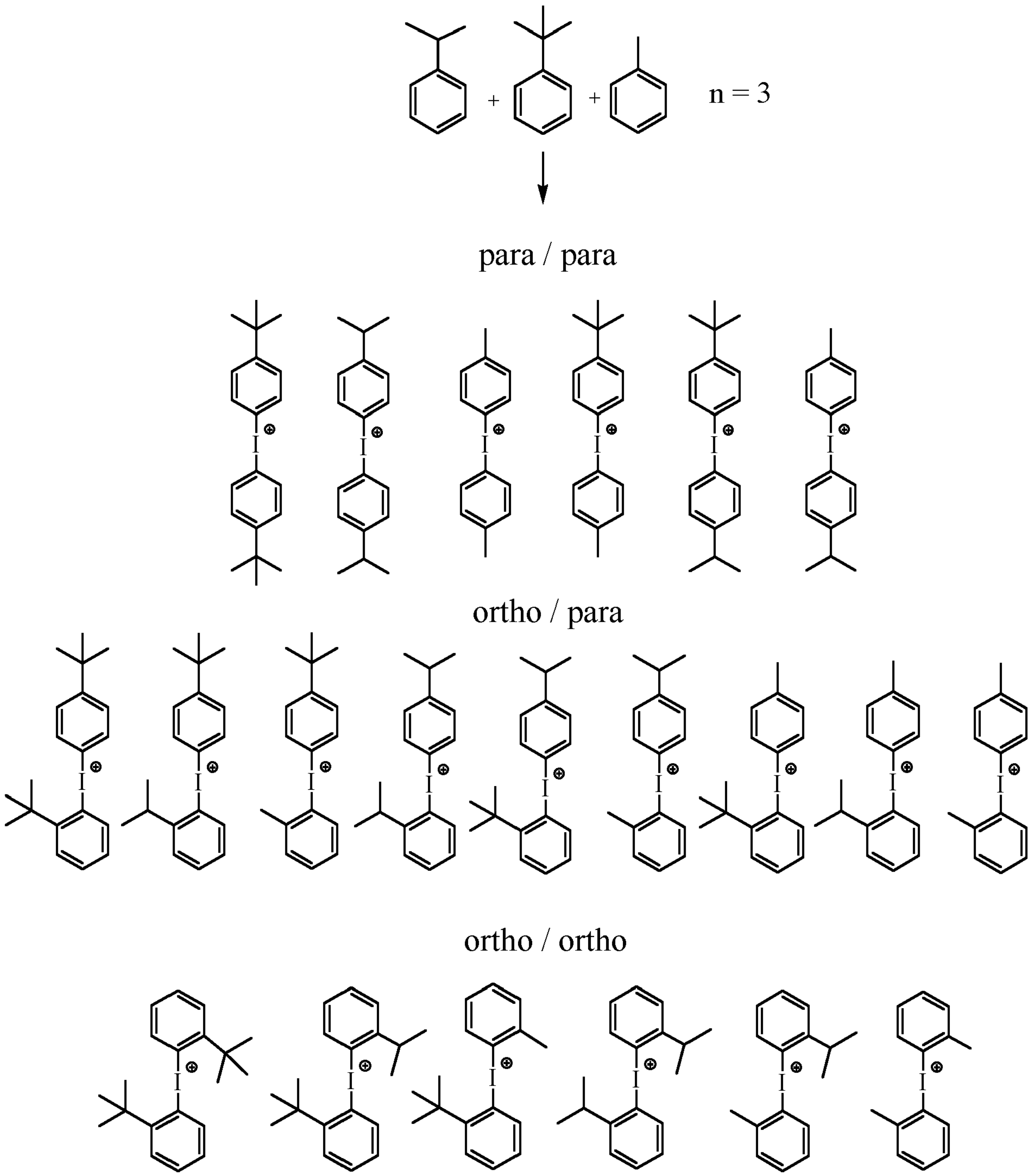

# Figure 2

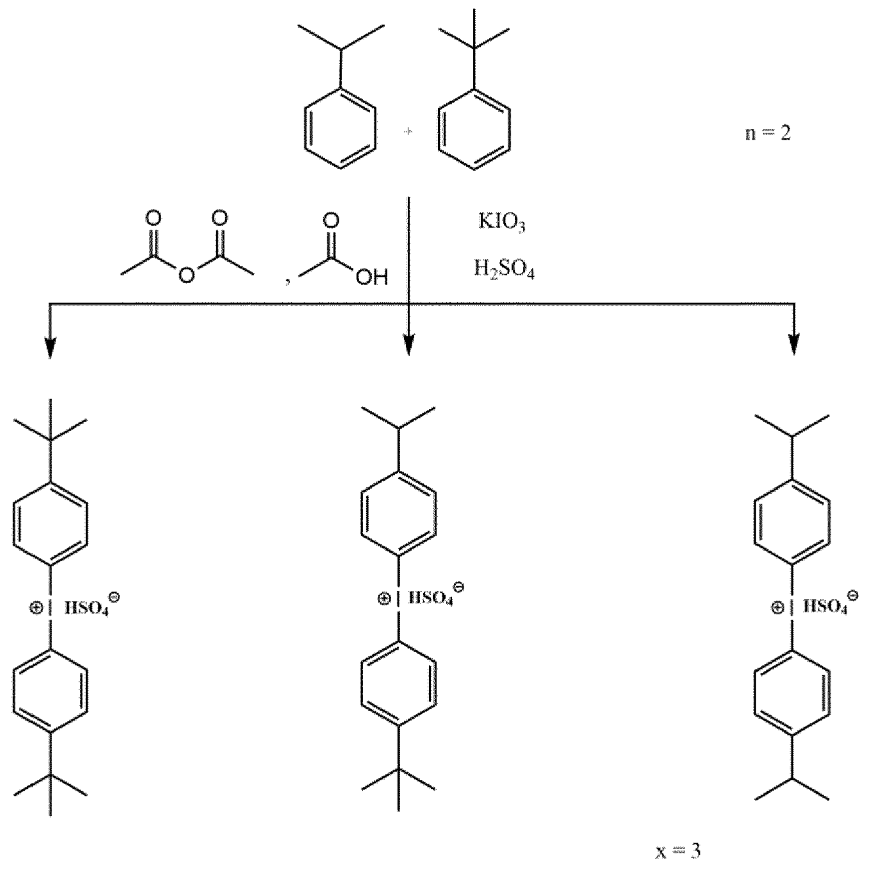

# Figure 3

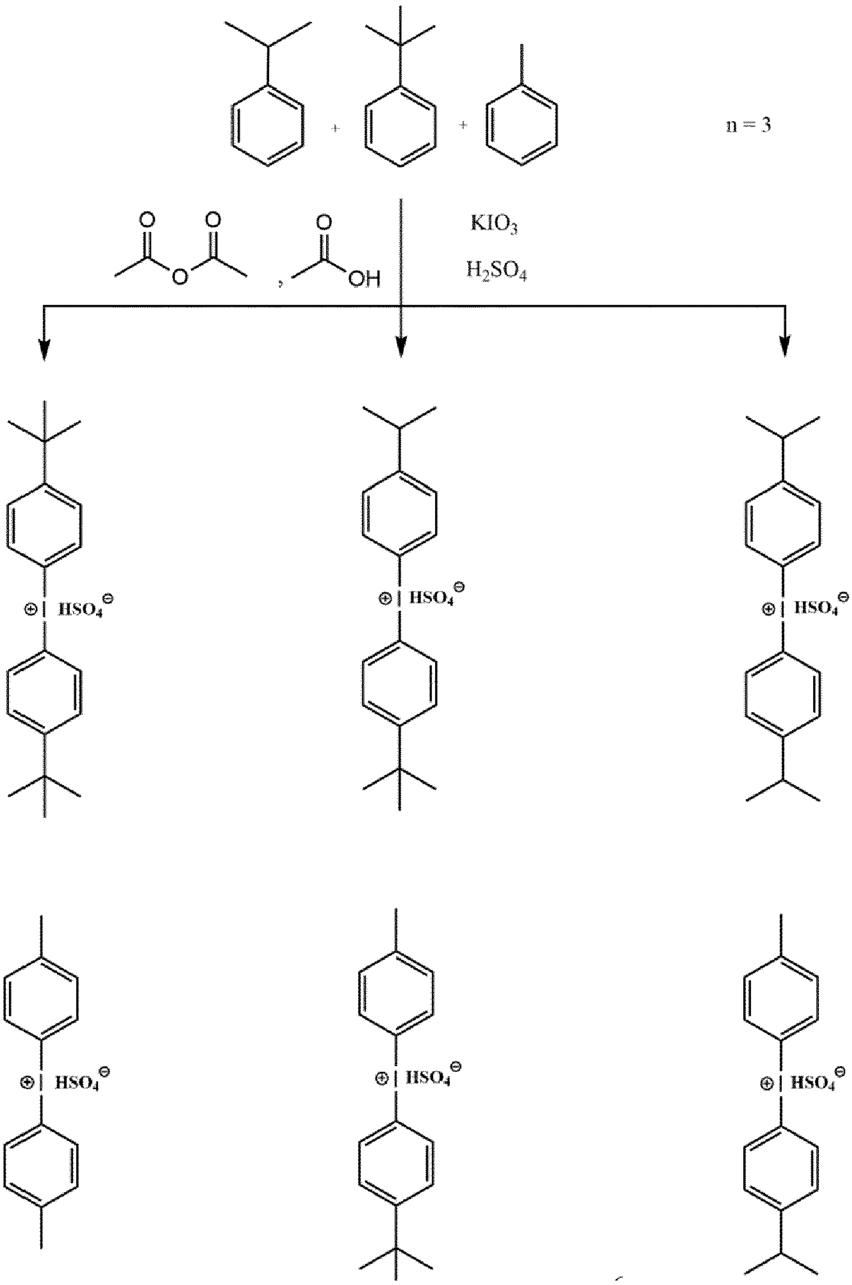

# Figure 4

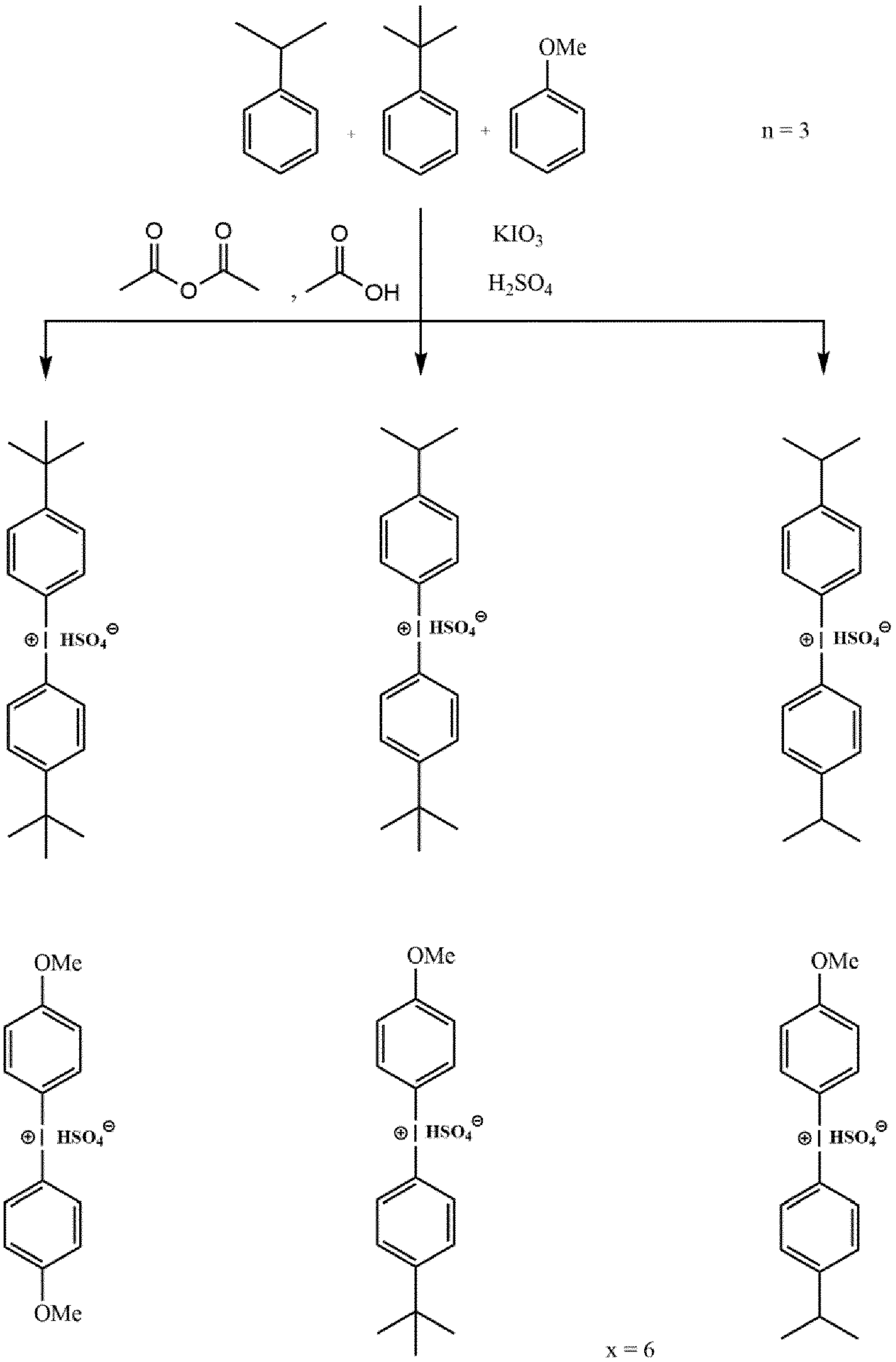

# Figure 5

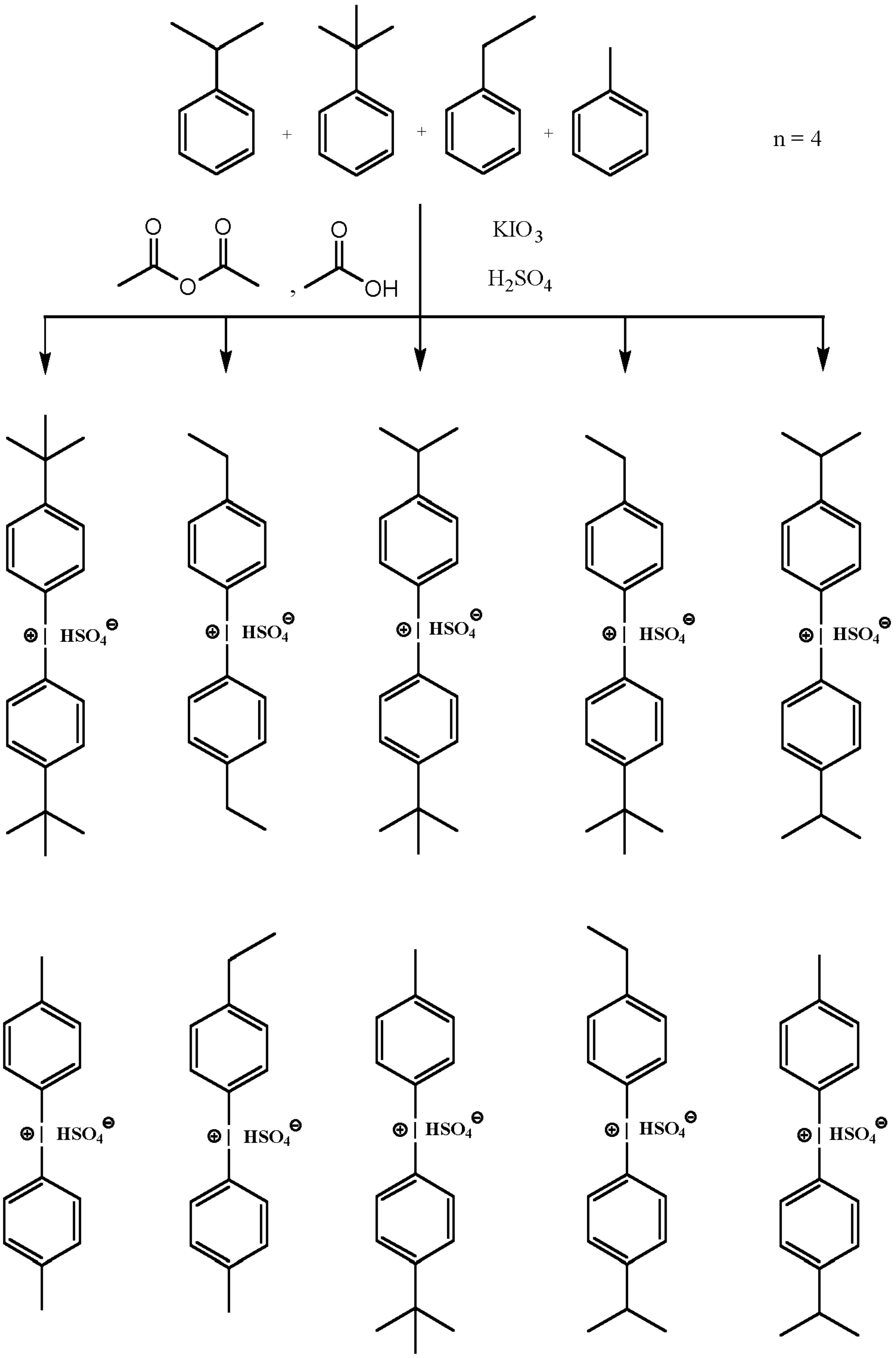


n = 4

x = 10

# Figure 6

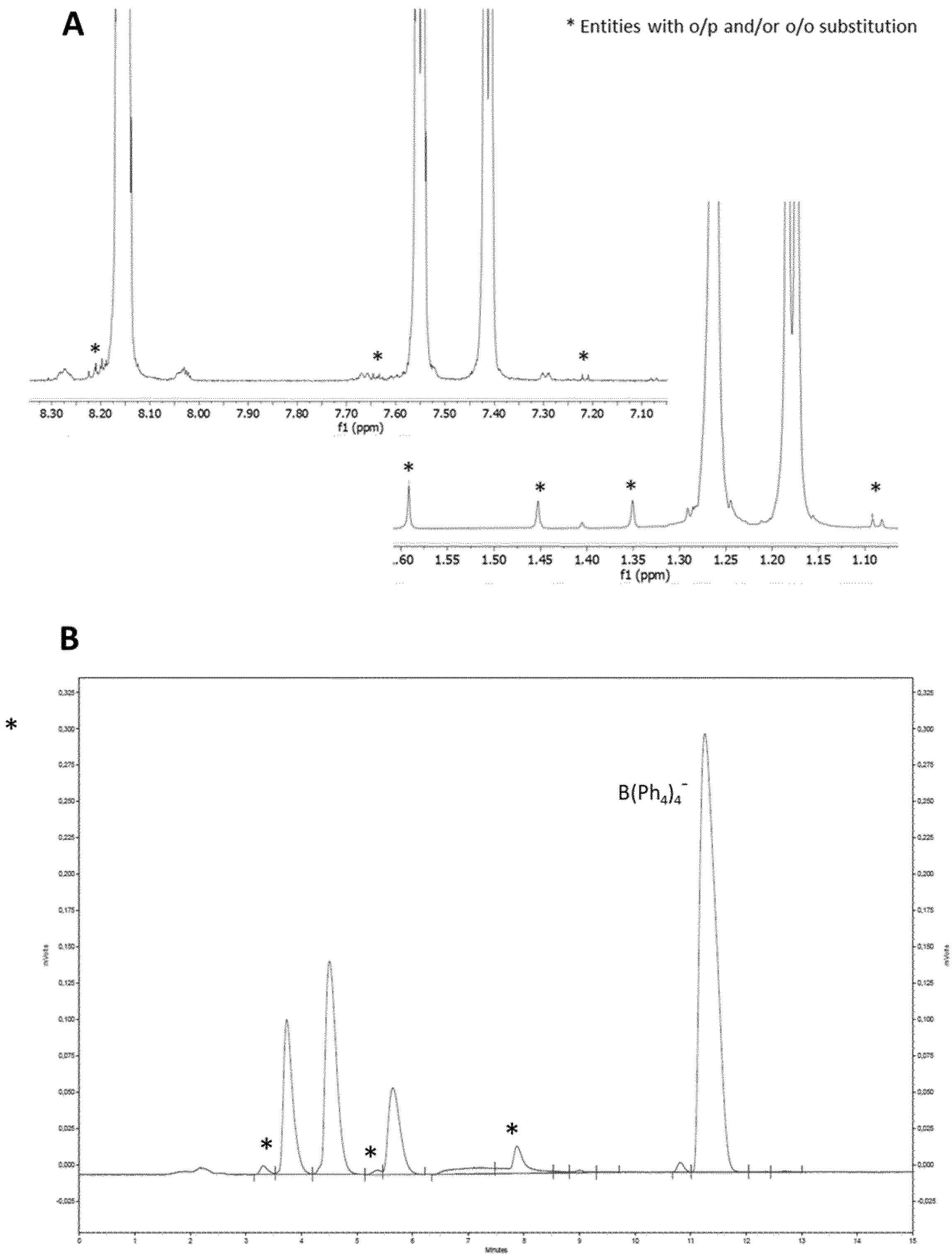
A
* Entities with o/p and/or o/o substitution
8.30 8.20 8.10 8.00 7.90 7.80 7.70 7.60 7.50 7.40 7.30 7.20 7.10
f1 (ppm)
.60 1.55 1.50 1.45 1.40 1.35 1.30 1.25 1.20 1.15 1.10
f1 (ppm)
B
$B(Ph_4)_4^-$
Minutes

# Figure 7

A

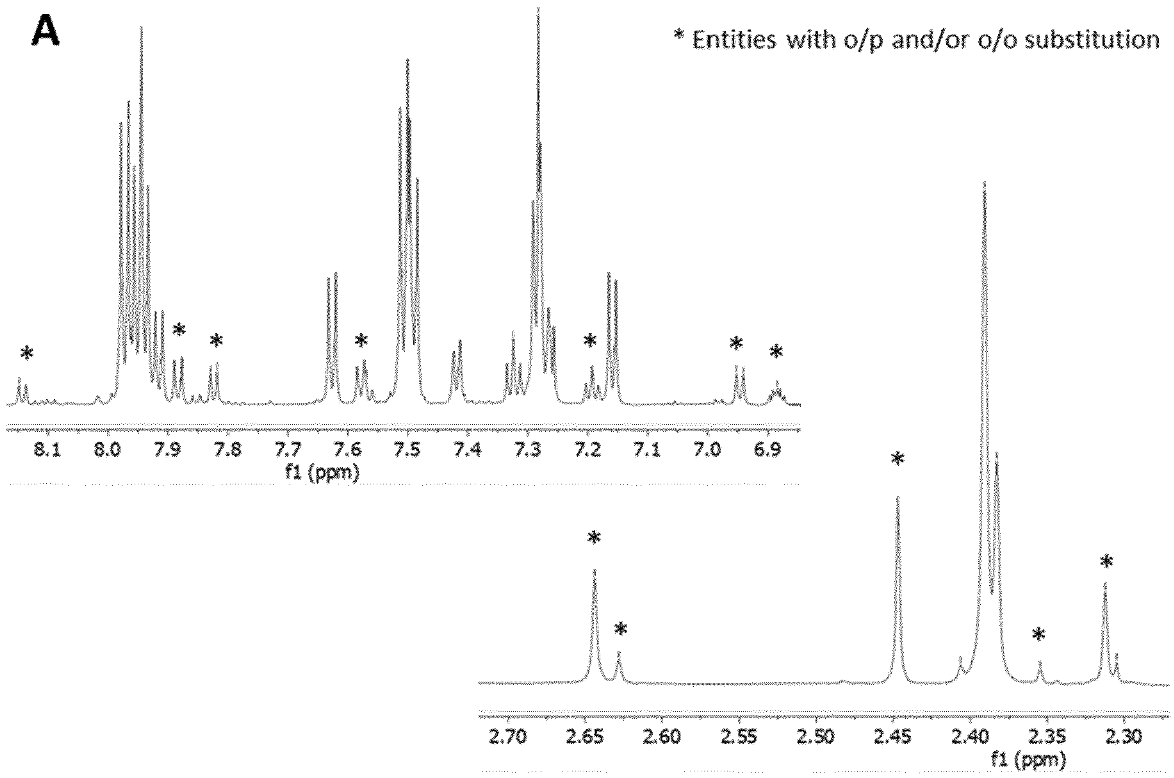
* Entities with o/p and/or o/o substitution
8.1
8.0
7.9
7.8
7.7
7.6
7.5
7.4
7.3
7.2
7.1
7.0
6.9
f1 (ppm)
2.70
2.65
2.60
2.55
2.50
2.45
2.40
2.35
2.30
f1 (ppm)

B

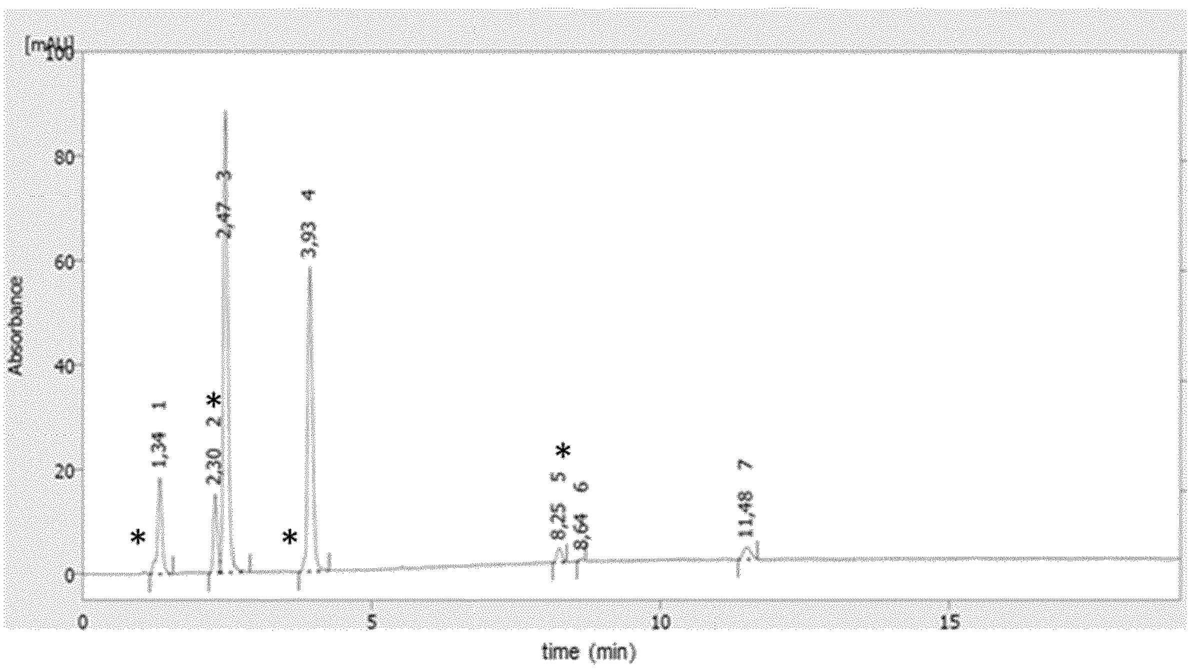
[mAU]
100
80
60
40
20
0
Absorbance
1,34 1
2,30 2
2,47 3
3,93 4
8,25 5
8,64 6
11,48 7
0
5
10
15
time (min)

Europäisches Patentamt
European Patent Office
Office européen des brevets

# EUROPEAN SEARCH REPORT

Application Number
EP 21 17 8716

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2017/131959 A1 (EASTMAN KODAK CO [US]) 3 August 2017 (2017-08-03)<br>* page 6, line 1 - line 5; claim 1; example 3 *<br>* page 18, line 13 - line 19 *<br>* page 17, line 13 - line 32 *<br>* page 13, line 1 - line 24 *<br>----- | 1-8, 11-14 | INV.<br>C07C25/18<br>C07C43/225<br>C08F2/48<br>C09D163/00<br><br>ADD.<br>G03F7/004<br>G03F7/029<br>B41C1/10 |
| X | MARSHALL BERINGER F ET AL: "Diaryliodonium salts", JOURNAL OF THE AMERICAN CHEMICAL SOCIETY, AMERICAN CHEMICAL SOCIETY, vol. 81, no. 2, 20 January 1959 (1959-01-20), pages 342-351, XP002109864, ISSN: 0002-7863, DOI: 10.1021/JA01511A020<br>* page 343, paragraph (B) *<br>----- | 9,10 | |

| TECHNICAL FIELDS SEARCHED (IPC) |
|---|
| C07C |
| G03F |
| B41C |
| C08F |

The present search report has been drawn up for all claims

1

| Place of search | Date of completion of the search | Examiner |
|---|---|---|
| The Hague | 25 November 2021 | Lacombe, Céline |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 17 8716

This annex lists the patent family members relating to the patent documents cited in the above-mentioned European search report.
The members are as contained in the European Patent Office EDP file on
The European Patent Office is in no way liable for these particulars which are merely given for the purpose of information.

25-11-2021

| Patent document cited in search report | Publication date | Patent family member(s) | Publication date |
|---|---|---|---|
| WO 2017131959 A1 | 03-08-2017 | CN 108602344 A | 28-09-2018 |
| | | EP 3408098 A1 | 05-12-2018 |
| | | JP 6853262 B2 | 31-03-2021 |
| | | JP 2019504780 A | 21-02-2019 |
| | | US 2017217149 A1 | 03-08-2017 |
| | | WO 2017131959 A1 | 03-08-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017131959 A
- WO 201711959 A1
- WO 2010128649 A1
- EP 2428501 A
- JP 5485583 B
- WO 20100128649 A
- EP 2428501 A1
- US 6899994 B, Huang
- JP 2015202586 A, Kamiya
- US 7261998 B, Hayashi
- US 20150099229, Simpson
- US 6916595 B, Fujimaki
- EP 1182033 A1, Fujimaki
- US 6309792 B, Hauck
- US 6569603 B, Furukawa
- US 6893797 B, Munnelly
- US 20090142695, Baumann
- US 7429445 B, Munnelly
- US 5208135 A, Patel
- US 6153356 A, Urano
- US 6264920 B, Achilefu
- US 6787281 B, Tao
- US 7018775 B, Tao
- US 7135271 B, Kawaushi
- WO 2004101280 A, Munnelly
- US 6858374 B, Yanaka
- US 64287615, Hayakawa
- US 8383319 B, Huang
- US 8105751 B, Endo
- US 20130323643, Balbinot
- WO 2017035552 A
- EP 1765593 A


### Non-patent literature cited in the description


- Berichte der Deutschen Chemischen Gesellschaft. Hartmann, vol. 27, 426
- **SMITH, WILLIAM F. ; HASHEMI, JAVAD.** Foundations of Materials Science and Engineering. Mc-Graw-Hill, 2006
- **H. J. LUCAS.** *Org. Synth.,* 1942, vol. 22, 52
- **R. B. SANDIN.** *Chem. Rev.,* 1943, vol. 32, 249
- **F. M. BERINGER et al.** *J. Am. Chem. Soc.,* 1959, vol. 81, 342
- *J. AM. CHEM. SOC.,* 1953, vol. 75, 2705
- **J. V. CRIVELLO ; J. H. W. LAM.** *J. Org. Chem.,* 1978, vol. 43, 3055
- **F. M. BERINGER et al.** *J. Am. Chem. Soc.,* 1958, vol. 80, 4279
- **Y. YAMADA ; M. OKAWARA.** *Bull. Chem. Soc. Jpn.,* 1972, vol. 45, 2515
- **G. F. KOSER et al.** *J. Org. Chem.,* 1980, vol. 45, 1543
- **C. S. CARMAN et al.** *J. Org. Chem.,* 1983, vol. 48, 2534
- **A. J. MARGIDA et al.** *J. Org. Chem.,* 1984, vol. 49, 3643
- **H. TOHMA et al.** *Angew. Chem.,* 2004, vol. 116, 3679
- *Angew. Chem. Int. Ed.,* 2004, vol. 43, 3595
- **T. DOHI et al.** *Angew. Chem.,* 2005, vol. 117, 6349
- *Angew. Chem. Int. Ed.,* 2005, vol. 44, 6193
- **M. OCHIAI et al.** *J. Am. Chem. Soc.,* 2005, vol. 127, 12244
- **M. BIELAWSKI et al.** *Chem. Commun.,* 2007, 2521
- *Adv. Synth. Catal.,* 2007, vol. 349, 2610
- **BRÖMME et al.** *Chem. Eng. Technol.,* 2016, vol. 39 (1), 13-25
- Imaging Technology, 3. Imaging in Graphic Arts. **BAUMANN et al.** Ullmann's Encyclopedia of Industrial Chemistry. Wiley-VCH Verlag GmbH & Co, 2015
- Radiation Curing in Polymer Science and Technology. **DUFOUR, P.** Fundamentals and Methods. Elsevier Science Publishers, 1993, vol. I, 1-28
- **SANGERMANO et al.** *Polymers,* 2018, vol. 10, 136
- **QUAN H. et al.** *Bioactive Materials,* 2020, vol. 5, 110-115
- **MALALLAH R. et al.** *Polymers,* 2017, vol. 9, 337
- Glossary of Basic Terms in Polymer Science. Pure Appl. Chem. International Union of Pure and Applied Chemistry (''IUPAC), 1996, vol. 68, 2287-2311
- **A REISER.** The Science and Technology of Resists. Wiley, 1989, 102-177
- **B.M. MONROE.** Radiation Curing: Science and Technology. Plenum, 1992, 399-440

- Polymer Imaging. **A.B. COHEN ; P. WALKER et al.** Imaging Processes and Material. Van Nostrand Reinhold, 1989, 226-262
- **SANGERMANO M.** *Macromolecular Materials and Engineering,* 2014, vol. 299 (7), 775-793
- Experiments in Green and Sustainable Chemistry. Wiley VCF, 2009, 13 1-144
- **R.B. FIGUEIRA et al.** *Journal of Coatings Technology and Research,* 2015, vol. 12, 1-35